# EUROPEAN PATENT APPLICATION

(11) **EP 3 838 404 A1**
(43) Date of publication of application: **23.06.2021**
(21) Application number: 21156490.1
(22) Date of filing: 28.04.2018
(51) Int. Cl.: B01J 38/18, C07C 5/333, B01J 19/26, C07C 5/32, B01J 4/00, B01J 8/02

(54) **APPARATUS FOR INTRODUCING GAS INTO A REACTOR**

(30) Priority: 28.04.2017 US 201762491688 P
(62) Divisional of application: 18726222.5
(71) Applicant: Flint Hills Resources, LP, Wichita, Kansas 67220 (US)
(72) Inventor: MAURO DE FEO, Demian, Wichita, Kansas 67220 (US); CLANCY-JUNDT, Brian, Wichita, Kansas 67220 (US)
(74) Representative: Ter Meer Steinmeister & Partner

(57) **Abstract**

A method for producing a dehydrogenated product and a coked catalyst, then introducing an oxygen-containing fluid, combusting at least a portion of the coke disposed on the catalyst in the presence of the oxygen-containing fluid to produce a decoked catalyst. An apparatus for introducing fluid into a reactor, comprising a first inlet conduit configured to convey a first gas, a second inlet conduit configured to convey a second gas, and an outlet conduit configured to convey the first gas and the second gas into a reactor, wherein there is an acute angle between a longitudinal axes of the first inlet conduit and a longitudinal axis of the second inlet conduit and an obtuse angle between a longitudinal axis of the outlet conduit and the longitudinal axis of the second inlet conduit and a pre-distributor disposed, in one embodiment on the inner surface, within the first inlet conduit is disclosed.

## Description

### CROSS-REFERENCE TO RELATED APPLICATIONS

This application claims priority to U.S. Provisional Patent Application No. 62/491,688 filed on April 28, 2017, herein incorporated by reference in its entirety.

### FIELD OF THE DISCLOSURE

Embodiments described generally relate to processes and apparatus for introducing a gas into a reactor. More particularly, such embodiments relate to processes and inlet assemblies for introducing a gas into a reactor having an improved velocity centroid.

### BACKGROUND

A commercially important refinery processes is the dehydrogenation of alkanes, *e.g*., propane, to produce the corresponding olefin or alkene, *e.g.*, propylene. One process that is widely used for the dehydrogenation of C₃ and C₄ alkanes is the CATOFIN® process. This process is a cyclical operation that cycles between a dehydrogenation mode and an air regeneration mode. One challenge in operating the dehydrogenation reactor is that the large reactor size makes it difficult to achieve a uniform distribution of gases within a catalyst bed disposed within the reactor. Improving the gas distribution into the catalyst bed is desirable because a more uniform gas flow results in more of the catalyst within the reactor being used to convert the alkane to the alkene.

Many devices have been developed that are intended to more uniformly distribute the gas flow within the reactor. These devices can generally be categorized as gas impingement structures and gas distribution structures that either redirect or direct the gas flow, respectively. The current devices, while providing some improvement in the distribution of gases introduced into the reactor, leave a considerable amount of room for improvement in achieving a uniform gas flow into the catalyst bed.

There is a need, therefore, for improved processes and inlet assemblies for introducing a gas into a dehydrogenation reactor.

### SUMMARY OF THE INVENTION

Processes and apparatus for introducing a gas into a reactor are provided. In some examples, a process for dehydrogenating an alkane can include introducing an alkane into a reactor from an outlet conduit of an inlet assembly. A catalyst within the reactor can be contacted with the alkane to produce a dehydrogenated product that can include an alkene and a coked catalyst that can include coke disposed on the catalyst. The dehydrogenated product can be separated from the coked catalyst. An oxygen-containing fluid can be introduced into the reactor from the outlet conduit. The oxygen-containing fluid can have a velocity profile at an end of the outlet conduit coupled to the reactor. The end of the outlet conduit coupled to the reactor can have a radial distance from an inner surface thereof to a center thereof. A velocity maxima of the velocity profile at the end of the outlet conduit coupled to the reactor can be at least 80% of the radial distance away from the inner surface thereof when a velocity of the oxygen-containing fluid is greater than or equal to 100 m/s. At least a portion of the coke disposed on the catalyst can be combusted in the presence of the oxygen-containing fluid to produce a decoked catalyst.

In some examples, a process for dehydrogenating an alkane can include introducing an alkane into a reactor from an outlet conduit of an inlet assembly. A catalyst within the reactor can be contacted with the alkane to produce a dehydrogenated product that can include an alkene and a coked catalyst that can include coke disposed on the catalyst. The dehydrogenated product can be separated from the coked catalyst. An oxygen-containing fluid can be introduced into the reactor from the outlet conduit. The oxygen-containing fluid can have a velocity profile at an end of the outlet conduit coupled to the reactor. The end of the outlet conduit coupled to the reactor can have a radial distance from an inner surface thereof to a center thereof. A velocity maxima of the velocity profile at the end of the outlet conduit coupled to the reactor can be at least 80% of the radial distance away from the inner surface thereof when a velocity of the oxygen-containing fluid is greater than or equal to 100 m/s, as determined using computational fluid dynamics modeling. At least a portion of the coke disposed on the catalyst can be combusted in the presence of the oxygen-containing fluid to produce a decoked catalyst.

In some examples, a process for regenerating a coked catalyst in a reactor can include flowing an oxygen-containing fluid through a first inlet conduit into a junction of an inlet assembly. A second inlet conduit and an outlet conduit can be coupled to the junction. There can be an acute angle between a longitudinal axis of the second inlet conduit and a longitudinal axis of the first inlet conduit. There can be an obtuse angle between the longitudinal axis of the second inlet conduit and a longitudinal axis of the outlet conduit. The oxygen-containing fluid can flow through the junction and into the outlet conduit. The oxygen-containing fluid can flow through the outlet conduit and into a reactor coupled to an end of the outlet conduit. The oxygen-containing fluid can have a velocity profile at the end of the outlet conduit coupled to the reactor. The end of the outlet conduit coupled to the reactor can have a radial distance from an inner surface thereof to a center thereof. A velocity maxima of the velocity profile at the end of the outlet conduit coupled to the reactor can be at least 80% of the radial distance away from the inner surface of the outlet conduit when a velocity of the oxygen-containing fluid is greater than or equal to 100 m/s. The reactor can include a coked catalyst that can include coke disposed on a catalyst. At least a portion of the coke disposed on the catalyst can be combusted in the presence of the oxygen-containing fluid to produce a regenerated catalyst.

In some examples, a process for regenerating a coked catalyst in a reactor can include flowing an oxygen-containing fluid through a first inlet conduit into a junction of an inlet assembly. A second inlet conduit and an outlet conduit can be coupled to the junction. There can be an acute angle between a longitudinal axis of the second inlet conduit and a longitudinal axis of the first inlet conduit. There can be an obtuse angle between the longitudinal axis of the second inlet conduit and a longitudinal axis of the outlet conduit. The oxygen-containing fluid can flow through the junction and into the outlet conduit. The oxygen-containing fluid can flow through the outlet conduit and into a reactor coupled to an end of the outlet conduit. The oxygen-containing fluid can have a velocity profile at the end of the outlet conduit coupled to the reactor. The end of the outlet conduit coupled to the reactor can have a radial distance from an inner surface thereof to a center thereof. A velocity maxima of the velocity profile at the end of the outlet conduit coupled to the reactor can be at least 80% of the radial distance away from the inner surface of the outlet conduit when a velocity of the oxygen-containing fluid is greater than or equal to 100 m/s, as determined using computational fluid dynamics modeling. The reactor can include a coked catalyst that can include coke disposed on a catalyst. At least a portion of the coke disposed on the catalyst can be combusted in the presence of the oxygen-containing fluid to produce a regenerated catalyst.

In some examples, a process for introducing a gas into a reactor can include flowing a gas through a first inlet conduit into a junction of an inlet assembly. A second inlet conduit and an outlet conduit can be coupled to the junction. There can be an acute angle between a longitudinal axis of the second inlet conduit and a longitudinal axis of the first inlet conduit. There can be an obtuse angle between the longitudinal axis of the second inlet conduit and a longitudinal axis of the outlet conduit. The gas can flow through the junction and into the outlet conduit. The gas can flow into a reactor through an end of the outlet conduit. The gas can have a velocity profile at the end of the outlet conduit. The end of the outlet conduit can have a radial distance from an inner surface thereof to a center thereof. A velocity maxima of the velocity profile at the end of the outlet conduit can be at least 80% of the radial distance away from the inner surface of the end of the outlet conduit when a velocity of the gas is greater than or equal to 100 m/s.

In some examples, an inlet assembly for introducing a fluid into a reactor can include a first inlet conduit, a second inlet conduit, and an outlet conduit fluidly connected at a junction. The first inlet conduit can be configured to convey a first gas therethrough, the second inlet conduit can be configured to convey a second gas therethrough, and the outlet conduit can be configured to convey the first gas and the second gas therethrough and into a reactor. There can be an acute angle between a longitudinal axes of the first inlet conduit and a longitudinal axis of the second inlet conduit. There can be an obtuse angle between a longitudinal axis of the outlet conduit and the longitudinal axis of the second inlet conduit. A pre-distributor can be disposed within the first inlet conduit.

In some examples, an inlet assembly for introducing a fluid into a reactor can include a first inlet conduit, a second inlet conduit, and an outlet conduit fluidly connected at a junction. The first inlet conduit can be configured to convey a first gas therethrough, the second inlet conduit can be configured to convey a second gas therethrough, and the outlet conduit can be configured to convey the first gas and the second gas therethrough and into a reactor. There can be an acute angle between a longitudinal axes of the first inlet conduit and a longitudinal axis of the second inlet conduit. There can be an obtuse angle between a longitudinal axis of the outlet conduit and the longitudinal axis of the second inlet conduit. A pre-distributor can be disposed on an inner surface of the first inlet conduit.

### BRIEF DESCRIPTION OF THE DRAWINGS

So that the manner in which the above recited features can be understood in detail, a more particular description, briefly summarized above, may be had by reference to embodiments, some of which are illustrated in the appended drawing. It is to be noted, however, that the appended drawing illustrate only typical embodiments and are therefore not to be considered limiting of its scope, for the invention may admit to other equally effective embodiments.
Figure 1 depicts a cross-sectional elevational view of an illustrative inlet assembly for introducing one or more gases into a rector that includes a pre-distributor, according to one or more embodiments described.
Figures 2-4 depict plan views of illustrative pre-distributors, according to one or more embodiments described.
Figure 5 depicts a cross-sectional elevational view of another illustrative inlet assembly for introducing one or more gases into a reactor that includes a first pre-distributor and a second pre-distributor, according to one or more embodiments described.
Figure 6 depicts a cross-sectional elevational view of an illustrative reactor for dehydrogenating one or more alkanes that includes the inlet assembly depicted in Figure 5, according to one or more embodiments described..
Figure 7 depicts a Computational Fluid Dynamics (CFD) inlet assembly model that is used in the CFD modeling of both large and small sized inlet assemblies in Examples C1, C2, and Ex. 1-4.
Figure 8 indicates the dimensions listed in Table 2 for both the large and small sized inlet assemblies of Examples C1, C2, and Ex. 1-4.
Figure 9 illustrates the distances AA, BB, and CC provided in Table 3.
Figures 10 and 11 illustrate the distances AA, BB, CC, and DD provided in Table 4.
Figures 12, 13, and 14 depict simulated air velocity contours through the inlet assemblies along the ZX-plane in Examples C1, Ex. 1, and Ex. 2, respectively.
Figures 15, 16, and 17 depict simulated air velocity contours through the inlet assemblies along the YZ-plane in Examples C1, Ex. 1, and Ex. 2, respectively.
Figures 18, 19, and 20 depict simulated air velocity fields at an end of outlet conduits of the inlet assemblies in Examples C1, Ex. 1, and Ex. 2, respectively.
Figures 21, 22, and 23 depict an approximate 3D-structure of simulated velocity profiles at the end of the outlet conduits of the inlet assemblies in Examples C1, Ex. 1, and Ex. 2, respectively.
Figure 24 depicts a coordinate position of a simulated velocity profile maxima at the end of the outlet conduits of the inlet assemblies in Examples C1, Ex. 1, and Ex. 2, respectively.
Figures 25, 26, and 27 depict simulated air velocity contours through the inlet assemblies along the ZX-plane in Examples C2, Ex. 3, and Ex. 4, respectively.
Figures 28, 29, and 30 depict simulated air velocity contours through the inlet assemblies along the YZ-plane of Examples C2, Ex. 3, and Ex. 4, respectively.
Figures 31, 32, and 33 depict simulated air velocity fields at an end of outlet conduits of the inlet assemblies in Examples C2, Ex. 3, and Ex. 4, respectively.
Figures 34, 35, and 36 depict an approximate 3D-structure of simulated velocity profiles at the end of the outlet conduits of the inlet assemblies in Examples C2, Ex. 3, and Ex. 4, respectively.
Figure 37 depicts a coordinate position of a simulated velocity profile maxima at the end of the outlet conduits of the inlet assemblies in Examples C2, Ex. 3, and Ex. 4, respectively.

### DETAILED DESCRIPTION OF THE INVENTION

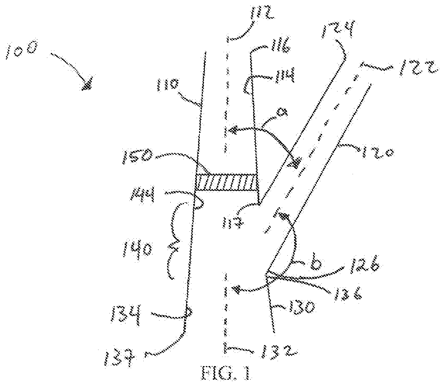

Figure 1 depicts a cross-sectional elevational view of an illustrative inlet assembly 100 for introducing one or more gases into a rector, *e.g.,* a dehydrogenation reactor, which includes a pre-distributor 150, according to one or more embodiments. As shown in Figure 1, the inlet assembly 100 can include a first inlet conduit or "first conduit" 110, a second inlet conduit or "second conduit" 120, and an outlet conduit 130 that can be fluidly connected at a junction 140. There can be an acute angle (a) between a central longitudinal axis 112 of the first conduit 110 and a central longitudinal axis 122 of the second conduit 120. There can be an obtuse angle (b) between the central longitudinal axis 122 of the second conduit 120 and a central longitudinal axis 132 of the outlet conduit 130. In some examples, the central longitudinal axis 112 of the first conduit 110 can be axially aligned with the central longitudinal axis 132 of the outlet conduit 130. It should be noted that the inlet assembly 100 is often referred to as a "cactus" assembly because of the branched conduits, *i.e.,* the first conduit 110 and the second conduit 120. The pre-distributor 150 can be disposed within the first conduit 110. In some examples, the pre-distributor 150 can be in physical contact with an inner surface 114 of the first conduit 110 about at least a portion of an inner perimeter thereof. In some examples, a gas tight seal can be formed between the pre-distributor 150 and the inner surface 114 of the first conduit 110.

Any number of pre-distributors 150 can be disposed within the first conduit. For example, the inlet assembly 100 can include 1, 2, 3, 4, 5, 6, 7, 8, 9, 10, or more pre-distributors 150 disposed within the first conduit 110. In at least one example, the inlet assembly 100 can include three pre-distributors 150. In at least one other example, the inlet assembly 100 can include one first pre-distributor 150.

It has been unexpectedly discovered that the inlet assembly used in a fixed bed dehydrogenation process, *e.g.,* the CATOFIN® dehydrogenation process, does not introduce the air, *e.g.,* during a catalyst regeneration cycle, uniformly into the reactor. Rather, the air significantly deviates from a central longitudinal axis of the inlet assembly, leading to a nonuniform distribution of air into the reactor. The inlet assembly generally includes at least two inlet conduits (often three or more inlet conduits) and an outlet conduit that are coupled to one another at a junction.

It has been surprisingly and unexpectedly discovered that placing, positioning, or otherwise locating the pre-distributor 150 within the first conduit 110 can significantly improve the distribution of a fluid, *e.g.,* a gas, introduced via the first conduit 110 into the inlet assembly 100 as the fluid exits from the outlet conduit 130. More particularly, it has been discovered that when the pre-distributor 150 is located within the first conduit 110, the centroid of a velocity profile of a fluid introduced via the first conduit 110 upon exiting the outlet 130 can be significantly more centralized with respect to the central longitudinal axis 132 of the outlet conduit 130 as compared to the same inlet assembly, but without the pre-distributor 150. Without wishing to be bound by theory, it is believed that in the absence of the pre-distributor 150 gas flow oscillations develop as the fluid, *e.g.,* air, flows from the first inlet conduit 110 through the junction 140 and into the outlet conduit 130. As the fluid flows through the inlet assembly 100, it is believed that a significant quantity of fluid flows along the inner surfaces 114, 144, and 134 of the first conduit 110, the junction 140, and the outlet conduit 130, respectively. It is also believed that eddies are formed that cause some of the fluid to circulate within the second conduit 115 that in turn cause flow oscillations of the fluid flowing through the inlet assembly 100. It is believed that these flow oscillations cause the centroid of the velocity profile to deviate from a central path through the inlet assembly 100 toward a side of the inlet assembly 100.

The pre-distributor 150 can provide an impingement surface along the inner surface 114 of the first inlet conduit 110 that can allow a majority of the fluid flow to pass through the junction while reducing or minimizing the formation of the eddies and improving the centroid of the velocity flow. The pre-distributor 150 can provide a significant benefit when the inlet assembly 100 is used to introduce a fluid, *e.g.,* an oxygen-containing fluid, into a reactor. For example, in the dehydrogenation of an alkane, *e.g.,* propane, to produce an alkene, *e.g.,* propylene, it has been found that the pre-distributor 150 can significantly improve the centroid of the velocity flow of an oxygen-containing fluid introduced via the first conduit 110 through the inlet assembly 100 and into the dehydrogenation reactor. Improving the centroid of the velocity flow of the oxygen-containing fluid can lead to a significant increase in the amount of alkene produced in the dehydrogenation process because improving the centroid of the velocity flow of the oxygen-containing fluid into the reactor can allow a more complete regeneration of the catalyst, which can provide more active catalyst sites for the dehydrogenation of the alkane. The fluid introduced via the inlet assembly 100 can be in the turbulent flow regime as the fluid flows through the inlet assembly 100.

The second end 137 of the outlet conduit 130 can have a radial distance from the inner surface 134 thereof to the center or central longitudinal axis 132 thereof. In some examples, the velocity maxima of the velocity profile of the oxygen-containing fluid at the second end 137 of the outlet conduit 130 can be at least 80%, at least 82%, at least 83%, at least 84%, at least 85%, at least 86%, at least 87%, at least 88%, at least 89%, at least 90%, at least 91%, at least 92%, at least 93%, at least 94%, at least 94.5%, at least 95%, at least 95.5%, at least 96%, at least 96.5%, at least 97%, at least 97.5%, or more of a radial distance away from the inner surface 134 thereof. In some examples, the velocity maxima of the velocity profile of the oxygen-containing fluid at the second end 137 of the outlet conduit 130 can be at least 80%, at least 82%, at least 83%, at least 84%, at least 85%, at least 86%, at least 87%, at least 88%, at least 89%, at least 90%, at least 91%, at least 92%, at least 93%, at least 94%, at least 94.5%, at least 95%, at least 95.5%, at least 96%, at least 96.5%, at least 97%, at least 97.5%, or more of a radial distance away from the inner surface 134 thereof when a velocity of the fluid, *e.g.,* an oxygen-containing fluid, is greater than or equal to 100 m/s. In some examples, the velocity maxima of the velocity profile of the oxygen-containing fluid at the second end 137 of the outlet conduit 130 can be at least 80%, at least 82%, at least 83%, at least 84%, at least 85%, at least 86%, at least 87%, at least 88%, at least 89%, at least 90%, at least 91%, at least 92%, at least 93%, at least 94%, at least 94.5%, at least 95%, at least 95.5%, at least 96%, at least 96.5%, at least 97%, at least 97.5%, or more of a radial distance away from the inner surface 134 thereof when a velocity of the fluid is greater than or equal to 150 m/s. In some examples, the velocity maxima of the velocity profile of the oxygen-containing fluid at the second end 137 of the outlet conduit 130 can be at least 80%, at least 82%, at least 83%, at least 84%, at least 85%, at least 86%, at least 87%, at least 88%, at least 89%, at least 90%, at least 91%, at least 92%, at least 93%, at least 94%, at least 94.5%, at least 95%, at least 95.5%, at least 96%, at least 96.5%, at least 97%, at least 97.5%, or more of a radial distance away from the inner surface 134 thereof when a velocity of the fluid is greater than or equal to 175 m/s. In some examples, the velocity maxima of the velocity profile of the oxygen-containing fluid at the second end 137 of the outlet conduit 130 can be at least 80%, at least 82%, at least 83%, at least 84%, at least 85%, at least 86%, at least 87%, at least 88%, at least 89%, at least 90%, at least 91%, at least 92%, at least 93%, at least 94%, at least 94.5%, at least 95%, at least 95.5%, at least 96%, at least 96.5%, at least 97%, at least 97.5%, or more of a radial distance away from the inner surface 134 thereof when a velocity of the fluid is greater than or equal to 200 m/s.

The addition of the pre-distributor 150 to the inlet assembly 100 can also significantly improve the velocity profile of the fluid not only at the second end 137 of the outlet conduit 130, but within the junction 140 and/or along the length of the outlet conduit 130. In some examples, the velocity maxima of the velocity profile of the fluid at the first end 136 of the outlet conduit 130 can be at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 94.5%, at least 95%, at least 95.5%, at least 96%, at least 96.5%, at least 97%, at least 97.5%, or more of a radial distance away from the inner surface 134 thereof. In some examples, the velocity maxima of the velocity profile of the fluid at the first end 136 of the outlet conduit 130 can be at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 94.5%, at least 95%, at least 95.5%, at least 96%, at least 96.5%, at least 97%, at least 97.5%, or more of a radial distance away from the inner surface 134 thereof when a velocity of the fluid is greater than or equal to 100 m/s. In some examples, the velocity maxima of the velocity profile of the fluid at the first end 136 of the outlet conduit 130 can be at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 94.5%, at least 95%, at least 95.5%, at least 96%, at least 96.5%, at least 97%, at least 97.5%, or more of a radial distance away from the inner surface 134 thereof when a velocity of the fluid is greater than or equal to 150 m/s. In some examples, the velocity maxima of the velocity profile of the fluid at the first end 136 of the outlet conduit 130 can be at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 94.5%, at least 95%, at least 95.5%, at least 96%, at least 96.5%, at least 97%, at least 97.5%, or more of a radial distance away from the inner surface 134 thereof when a velocity of the fluid is greater than or equal to 175 m/s. In some examples, the velocity maxima of the velocity profile of the fluid at the first end 136 of the outlet conduit 130 can be at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 94.5%, at least 95%, at least 95.5%, at least 96%, at least 96.5%, at least 97%, at least 97.5%, or more of a radial distance away from the inner surface 134 thereof when a velocity of the fluid is greater than or equal to 200 m/s.

In some examples, the velocity maxima of the velocity profile of the fluid at the second end 137 of the outlet conduit 130 and/or the velocity maxima of the velocity profile of the fluid at the first end 136 of the outlet conduit 130 can be estimated, computed, or otherwise determined via computational fluid dynamics (CFD) modeling. For example, the CFD modeling can use a CFD solver and a meshing solver. In some examples the CFD solver can be the Ansys CFX V17.2 and the meshing solver can be the Ansys Mesher V17.2. The CFD modeling can utilize a set of mesh details for geometry with no internals based on inlet diameter being equal to D_{I}. Illustrative mesh details can include, but are not limited to, a mesh type that can be fully tetrahedral with wall inflation; a size function refinement that can be proximity and curvature; a number of elements that can be greater than 1800000; a number of wall inflation layers that can be 7; a maximum face size that can be about 0.0800D; a minimum face sizing that can be about 0.0123D; and a maximum tetrahedral size that can be about 0.0984D.

Returning to the inlet assembly 100, the first conduit 110 can have a first average cross-sectional length, *e.g.,* a first diameter, at a first or "top" end 116 and a second average cross-sectional length, *e.g.,* a second diameter, at a second or "junction" end 117. In some examples, the first average cross-sectional length at the first end 116 and the second average cross-sectional length at the second end 117 can be the same. In other examples, the first average cross-sectional length at the first end 116 can be less than the second average cross-sectional length at the second end 117. For example, the first conduit 110 can have a frusto-conical inner surface. In some examples, however, the first conduit 110 can have a cylindrical inner surface or any other profile.

The second conduit 120 can have a first average cross-sectional length at a first or "top" end 124 and a second average cross-sectional length at a second or "junction" end 126 thereof. In some examples, the first average cross-sectional length at the first end 124 and the second average cross-sectional length at the second end 126 thereof can be the same. In other examples, the first average cross-sectional length at the first end 124 and the second average cross-sectional length at the second end 126 thereof can be different. In some examples, the second conduit 120 can have a cylindrical inner surface. In other examples, the second conduit 120 can have a frusto-conical inner surface.

The outlet conduit 130 can have a first average cross-sectional length, *e.g.,* first diameter, at a first or "junction" end 136 and a second average cross-sectional length, *e.g.,* second diameter, at a second or "outlet" end 137. In some examples, the first average cross-sectional length at the first end 136 and the second average cross-sectional length at the second end 137 can be the same. In other examples, the first average cross-sectional length of the first end 136 can be less than the second average cross-sectional length at the second end 137. For example, the outlet conduit 130 can have a frusto-conical inner surface. In some examples, however, the outlet conduit 130 can have a cylindrical inner surface or any other profile.

If the second average cross-sectional length at the second end 117 of the first conduit 110 is different than the first average cross-sectional length at the first end 136 of the outlet conduit 130, the inner surface 144 of the junction 140 can transition from the second average cross-sectional length at the second end 117 of the first conduit 110 to the first average cross-sectional length at first end 136 of the outlet conduit 130 in a substantially smooth manner. For example, if the first conduit 110 and the outlet conduit 130 have frusto-conical inner surfaces 114 and 134, respectively, then the junction 140 can also have a frusto-conical inner surface 144 to provide a substantially frusto-conical inner surface through the first conduit 110, the junction 140, and the outlet conduit 130.

The acute angle (a) between the central longitudinal axis 112 of the first conduit 110 and the central longitudinal axis 122 of the second conduit 120 can be about 10°, about 20°C, about 30°, or about 40° to about 50°, about 60°, about 70°, or about 80°. The obtuse angle (b) between the central longitudinal axis 122 of the second conduit 120 and the central longitudinal axis 132 of the outlet conduit can be about 100°, about 110°, about 120°, or about 130° to about 140°, about 150°, about 160°, or about 170°. In some examples, the acute angle (a) can be about 40° to about 50°, *e.g.,* about 45°, and the obtuse angle can be about 130° to about 140°, *e.g.,* about 135°.

The pre-distributor 150 can have any structure capable of directing or otherwise urging at least a portion of any gas flowing along the inner surface 114 of the first conduit 110 away from the inner surface 114 and toward the central longitudinal axis 112 of the first conduit 110. In some examples, the pre-distributor 150 can be or include a band of material. The band of material can be disposed on the inner surface 112 of the first conduit 110. For example, the band of material can be disposed about an inner perimeter of the first conduit 110. In some examples, the band of material can have a substantially symmetrical inner surface. In some examples, the band of material can be or include one or more rings having a substantially constant width and the external perimeter, *e.g*., outer circumference, of the ring can be attached, fixed, coupled, or otherwise secured to the inner surface 112 of the first conduit 110. In some examples, the band of material can be or include one or more rings having a substantially constant width and a plurality of tabs can be attached to an inner surface of the ring.

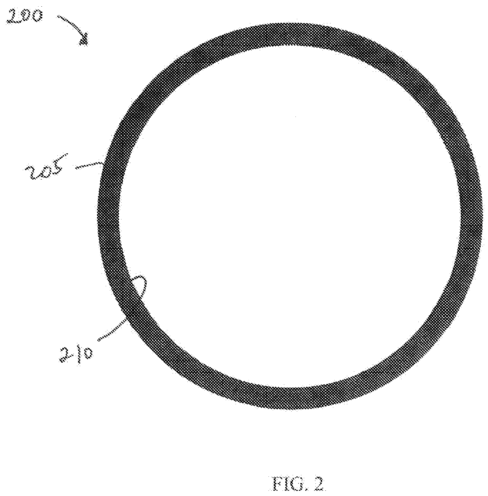

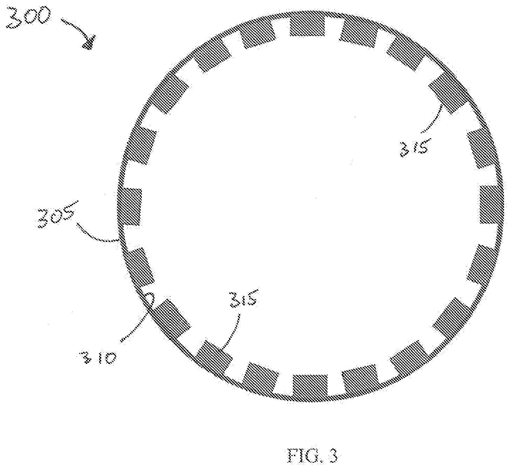

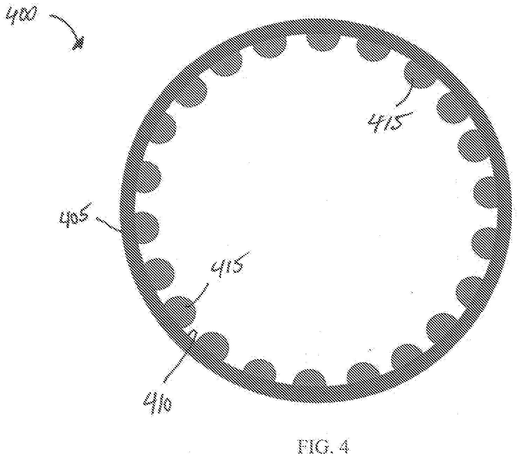

Figures 2-4 depict plan views of illustrative pre-distributors 200, 300, and 400, respectively, which can be disposed within the first conduit 110. As shown in Figure 2, the pre-distributor 200 can be a band of material having a form of a ring that can have a substantially constant width between an outer surface or perimeter 205 and an inner surface or perimeter 210 thereof. As shown in Figure 3, the pre-distributor 300 can be a band of material having a form of a ring that can have a substantially constant width between an outer surface or perimeter 305 and an inner surface or perimeter 310 thereof. The pre-distributor 300 can also include a plurality of rectangular or semi-rectangular tabs 315 secured to the inner surface or perimeter 310 thereof. The tabs 315 can be referred to as "semi-rectangular" rather than "rectangular" because the side attached to the inner surface 310 can be curved to generally correspond thereto. As shown in Figure 4, the pre-distributor 400 can be a band of material having a form of a ring that can have a substantially constant width between an outer surface or perimeter 405 and an inner surface or perimeter 410 thereof. The pre-distributor 400 can also include a plurality of semi-elliptical tabs 415 secured to the inner surface or perimeter 410 thereof. It should be noted that the each of the plurality of tabs 310 and 410 can have the same shape or a different shape with respect to one another. In at least one example, the plurality of tabs 310, 410 can provide a symmetrical inner surface or inner perimeter. In such an example, if two or more differently shaped tabs are used in the construction of the pre-distributor 300 and/or 400, tabs having the same or substantially the same shape can be disposed across from one another on the inner surfaces 315, 415 of the rings 305, 405, respectively, to provide a symmetrical inner surface.

The plurality of tabs 315, 415 can have any desired geometrical shape. Illustrative shapes can include, but are not limited to, the semi-rectangular and semi-elliptical shapes depicted in Figures 3 and 4. In addition to the semi-rectangular and semi-elliptical shapes, other geometrical shapes can include semi-triangular, semi-polygons having more than four sides such as a semi-pentagon, a semi-hexagon, a semi-heptagon, a semi-octagon, a semi-oval, a semi-parallelogram, a semi-trapezoid, a semi-kite, a semi-trapezium, a semi-crescent, or any combination thereof.

The pre-distributors 200, 300, and 400 can each have any desired thickness, *i.e.,* the length along the central longitudinal axis of the first conduit 110. For example, the pre-distributors 200, 300, and 400 can each have a thickness of about 5 mm, about 10 mm, about 20 mm, about 30 mm, about 40 mm, about 45 mm, or about 50 mm to about 55 mm, about 60 mm, about 70 mm, about 80 mm, about 90 mm, or about 100 mm. The pre-distributors 300 and 400 can include any number of tabs 310 and 410, respectively. For example, the pre-distributors 300 and 400 can include about 10, about 15, about 20, or about 25 to about 30, about 50, about 75, about 100 or more tabs 315 and 415, respectively, disposed about the inner surface or perimeter 310, 410, respectively.

The pre-distributors 200, 300, and 400 can each be made of any suitable material. In some examples, the pre-distributors 200, 300, and 400 can be made of a solid material, *e.g*., solid metal. In other examples, the pre-distributor can be made of a porous material, *e.g.,* a ceramic or sintered metal. Illustrative metals can include, but are not limited to, stainless steel, stainless steel alloys, carbon steel, carbon still alloys, nickel, nickel alloys, low chrome steel, or any combination thereof. In some examples, the metal can be or include a metal alloy that can include, but is not limited to, nickel, iron, chromium, cobalt, silicon, manganese, molybdenum, titanium, carbon, tungsten, and columbium. Illustrative ceramic materials can be or include, but are not limited to, silicon dioxide, aluminum oxide, titanium dioxide, magnesium oxide, or any combination thereof.

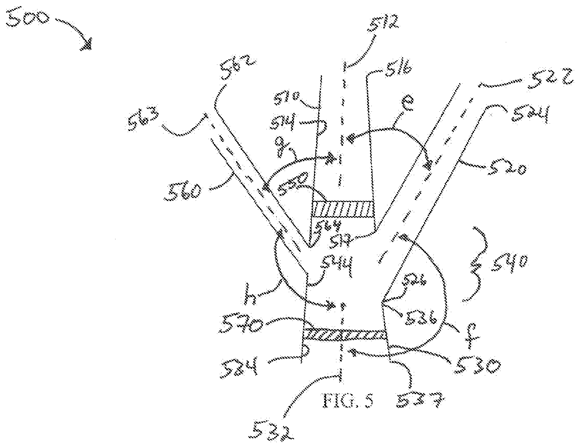

Figure 5 depicts a cross-sectional elevational view of another illustrative inlet assembly 500 for introducing one or more gases into a reactor, *e.g.,* a dehydrogenation reactor, which includes a first pre-distributor 550 and a second pre-distributor 570, according to one or more embodiments. As shown in Figure 5, the inlet assembly 500 can include a first inlet conduit or "first conduit" 510, a second inlet conduit or "second conduit" 520, a third inlet conduit or "third conduit" 560, and an outlet conduit 530 that can be fluidly connected at a junction 540. There can be an acute angle (e) between a central longitudinal axis 512 of the first conduit 510 and a central longitudinal axis 522 of the second conduit 520. There can be an obtuse angle (f) between the central longitudinal axis 522 of the second conduit 520 and a central longitudinal axis 532 of the outlet conduit 530. There can be an acute angle (g) between the central longitudinal axis 512 of the first conduit 510 and a central longitudinal axis 563 of the third conduit 560. There can be an obtuse angle (h) between the central longitudinal axis 563 of the third conduit 560 and the central longitudinal axis 532 of the outlet conduit 530. It should be noted that the inlet assembly 500, like the inlet assembly 100, is often referred to as a "cactus" assembly because of the branched conduits, *i.e.,* the first conduit 510, the second conduit 520, and the third conduit 560. In some examples, the central longitudinal axis 512 of the first conduit 510 can be axially aligned with the central longitudinal axis 532 of the outlet conduit 530.

The first pre-distributor 550 can be disposed within the first conduit 510 and the second pre-distributor 570 can be disposed within the outlet conduit 530. It should be noted that any number of first pre-distributors 550 can be disposed within the first conduit 210 and any number of second pre-distributors 570 can be disposed within the outlet conduit 530. For example, the inlet assembly 500 can include 1, 2, 3, 4, 5, 6, 7, 8, 9, 10, or more first pre-distributors 550 disposed within the first conduit 510 and 1, 2, 3, 4, 5, 6, 7, 8, 9, 10, or more second pre-distributors 570 disposed within the outlet conduit 530. In at least one example, the inlet assembly 500 can include three first pre-distributors 550 and two second pre-distributors 570. In at least one other example, the inlet assembly 500 can include one first pre-distributor 550 and two second pre-distributors 570.

The first conduit 510 can have a first average cross-sectional length, *e.g.,* a first diameter, at a first or "top" end 516 and a second average cross-sectional length, *e.g.,* a second diameter, at a second or "junction" end 517. In some examples, the first average cross-sectional length at the first end 516 and the second average cross-sectional length at the second end 517 can be the same. In other examples, the first average cross-sectional length at the first end 516 can be less than the second average cross-sectional length at the second end 517. For example, the first conduit 510 can have a frusto-conical inner surface. In some examples, however, the first conduit 510 can have a cylindrical inner surface or any other profile.

The second conduit 520 can have a first average cross-sectional length at a first or "top" end 524 and a second average cross-sectional length at a second or "junction" end 526 thereof. In some examples, the first average cross-sectional length at the first end 524 and the second average cross-sectional length at the second end 526 thereof can be the same. In other examples, the first average cross-sectional length at the first end 524 and the second average cross-sectional length at the second end 526 thereof can be different. In some examples, the second conduit 520 can have a cylindrical inner surface. In other examples, the second conduit 520 can have a frusto-conical inner surface.

The third conduit 560 can have a first average cross-sectional length at a first or "top" end 562 and a second average cross-sectional length at a second or "junction" end 564 thereof. In some examples, the first average cross-sectional length at the first end 562 and the second average cross-sectional length at the second end 564 thereof can be the same. In other examples, the first average cross-sectional length at the first end 562 and the second average cross-sectional length at the second end 564 thereof can be different. In some examples, the third conduit 560 can have a cylindrical inner surface. In other examples, the third conduit 560 can have a frusto-conical inner surface.

The outlet conduit 530 can have a first average cross-sectional length, *e.g.,* first diameter, at a first or "junction" end 536 and a second average cross-sectional length, *e.g.,* second diameter, at a second or "outlet" end 537. In some examples, the first average cross-sectional length at the first end 536 and the second average cross-sectional length at the second end 537 can be the same. In other examples, the first average cross-sectional length of the first end 536 can be less than the second average cross-sectional length at the second end 537. For example, the outlet conduit 530 can have a frusto-conical inner surface. In some examples, however, the outlet conduit 530 can have a cylindrical inner surface or any other profile.

If the second average cross-sectional length at the second end 517 of the first conduit 110 is different than the first average cross-sectional length at the first end 536 of the outlet conduit 530, the inner surface 544 of the junction 540 can transition from the second average cross-sectional length at the second end 517 of the first conduit 510 to the first average cross-sectional length at first end 536 of the outlet conduit 530 in a substantially smooth manner. For example, if the first conduit 510 and the outlet conduit 530 have frusto-conical inner surfaces 514 and 534, respectively, then the junction 540 can also have a frust-conical inner surface 544 to provide a substantially frusto-conical inner surface through the first conduit 510, the junction 540, and the outlet conduit 530.

The acute angle (e) between the central longitudinal axis 512 of the first conduit 510 and the central longitudinal axis 522 of the second conduit 520 can be about 10°, about 20°C, about 30°, or about 40° to about 50°, about 60°, about 70°, or about 80°. The obtuse angle (f) between the central longitudinal axis 522 of the second conduit 520 and the central longitudinal axis 532 of the outlet conduit can be about 100°, about 110°, about 120°, or about 130° to about 140°, about 150°, about 160°, or about 170°. The acute angle (g) between the central longitudinal axis 512 of the first conduit 510 and the central longitudinal axis 563 of the third conduit 560 can be about 10°, about 20°C, about 30°, or about 40° to about 50°, about 60°, about 70°, or about 80°. The obtuse angle (h) between the central longitudinal axis 563 of the third conduit 560 and the central longitudinal axis 532 of the outlet conduit can be about 100°, about 110°, about 120°, or about 130° to about 140°, about 150°, about 160°, or about 170°. In some examples, the acute angle (e) can be about 40° to about 50°, *e.g.,* about 45°, the obtuse angle (f) can be about 130° to about 140°, *e.g.,* about 135°, the acute angle (g) can be about 40° to about 50°, *e.g.,* about 45°, and the obtuse angle (g) can be about 130° to about 140°, *e.g.,* about 135°.

The first pre-distributor 550 and the second pre-distributor 570 can be the same or substantially similar to the pre-distributors 150, 200, 300, and/or 400 discussed and described above with reference to Figures 1-4. As such, the first pre-distributor 550 and the second pre-distributor can be or include a band of material, a plurality of tabs, or a combination thereof. It should be noted that any one of the pre-distributors can be composed of a plurality of tabs that can be directly attached to the inner surface of the first conduit 110 and/or 510. As such, the pre-distributors 150, 200, 300, 400, 550, and 570 can be free from any continuous band of material that traverses the inner perimeter of the first conduit 110 and/or 510.

The pre-distributors 150, 200, 300, 400, 550, and 570 can be coupled, secured, or otherwise attached to the inner surface 114, 514 of the first conduit 110, 510, respectively, via any suitable attachment device or combination of attachment devices. For example, the pre-distributors 150, 200, 300, 400, 550, and 570 can be welded, bolted, riveted, formed as an integral component of the first conduit, clamped, and/or held in place via friction fit. In some examples, the pre-distributors 150, 200, 300, 400, 550, and/or 570 can be a ledge or other surface that can be milled, machined, cut, pressed, or otherwise formed into the inner surface 114, 514 of the first conduits 110, 510, respectively, and/or the inner surface 134, 534 of the outlet conduits 130, 530, respectively.

Some of the dimensions of the inlet assemblies 100 and 500 can be described as a ratio of a given dimension to the average cross-sectional length of the second ends 137, 537 of the outlet conduits 130, 530, respectively. For ease of explanation, the average cross-sectional lengths of the various conduits can be described as being diameters of a circle. Accordingly, the "D" below refers to the inner diameter at the second ends 137, 537 of the outlet conduits 130, 530, respectively. It should be understood, however, that any one or more of these cross-sectional lengths can be non-circular, *e.g*., oval, a non-circular ellipse, a polygon, or any other geometrical shape.

Considering the inlet assemblies 100 and 500, the first ends 116, 516 of the first conduits 110, 510 can have a diameter of about 0.3D, about 0.33D, about 0.35D, about 0.37D, or about 0.4D to about 0.41D, about 0.43D, about 0.45D, about 0.47D, or about 0.5D. A distance from a center of the junctions 140, 540 to the second ends 137, 537 of the outlet conduits 130, 530, respectively, can be about 0.7D, about 0.75D, about 0.8D, about 0.85D, about 0.9D, or about 0.95D to about 0.97D, about ID, about 1.05D, about 1.1D, about 1.15D, about 1.2D about 1.25D, or about 1.3D. A distance from the first ends 116, 516 of the first conduits 110, 510 to the second ends 137, 537 of the outlet conduits 130, 530, respectively, can be about 1.7D, about 1.8D, about 1.9D, about 2D, about 2.1D, about 2.2D, about 2.3D, or about 2.4D to about 2.5D, about 2.6D, about 2.7D, about 2.8D, about 2.9D, about 3D, about 3. ID, or about 3.2D. The second conduits 120, 520 can have a cylindrical inner surface and can have an inner diameter of about 0.3D, about 0.33D, about 0.35D, about 0.37D, about 0.4D, or about 0.42D to about 0.44D, about 0.47D, about 0.5D, about 0.53D, or about 0.55D. The third conduit 560 can have a cylindrical inner surface and can have an inner diameter of about 0.15D, about 0.17D, about 0.19D, about 0.2D, or about 0.21D to about 0.22D, about 0.24D, about 0.26D, about 0.28D, or about 0.3D.

The components of the inlet assemblies 100 and 500, *i.e.,* the first conduits 110, 510, the second conduits 120, 520, the third conduit 560, the junctions 140, 540, and the outlet conduits 130, 530 can be made of any suitable material. In some examples the components of the inlet assemblies 100 and 500 can be made of one or more metals, one or more ceramics, or a combination thereof. Illustrative metals can include, but are not limited to, stainless steel, stainless steel alloys, carbon steel, carbon still alloys, nickel, nickel alloys, low chrome steel, or any combination thereof. In some examples, the metal can be or include a metal alloy that can include, but is not limited to, nickel, iron, chromium, cobalt, silicon, manganese, molybdenum, titanium, carbon, tungsten, and columbium. Illustrative ceramic materials can be or include, but are not limited to, silicon dioxide, aluminum oxide, titanium dioxide, magnesium oxide, or any combination thereof.

In some examples, one or more fluids, *e.g.,* air, can be introduced via the first conduit 510 and can flow through the inlet assembly 500. The fluid can have a velocity profile at the second end 537 of the outlet conduit 530. The second end 537 of the outlet conduit 530 can have a radial distance from the inner surface 534 thereof to the central longitudinal axis 532 thereof. In some examples, a velocity maxima of the velocity profile at the second end 537 of the outlet conduit 530 can be least 80% of the radial distance away from the inner surface 534 thereof. In some examples, the velocity maxima of the velocity profile at the second end 537 of the outlet conduit 530 can be least 82%, at least 84%, at least 86%, at least 88%, at least 90%, at least 92%, at least 93%, at least 94%, at least 94.5%, at least 95%, at least 95.5%, at least 96%, at least 96.5%, at least 97%, at least 97.5%, or more of the radial distance away from the inner surface 534 thereof. In some examples, the velocity maxima of the velocity profile of the oxygen-containing fluid at the second end 137 of the outlet conduit 130 can be at least 80%, at least 82%, at least 83%, at least 84%, at least 85%, at least 86%, at least 87%, at least 88%, at least 89%, at least 90%, at least 91%, at least 92%, at least 93%, at least 94%, at least 94.5%, at least 95%, at least 95.5%, at least 96%, at least 96.5%, at least 97%, at least 97.5%, or more of a radial distance away from the inner surface 134 thereof when a velocity of the fluid is greater than or equal to 100 m/s, 125 m/s, 150 m/s, 175 m/s, and/or 200 m/s. As noted above, the velocity maxima of the velocity profile can be measured, computed, determined, or otherwise estimated via computational fluid dynamics (CFD) modeling. For example, the CFD modeling can use a CFD solver and a meshing solver. In some examples the CFD solver can be the Ansys CFX V17.2 and the meshing solver can be the Ansys Mesher V17.2.

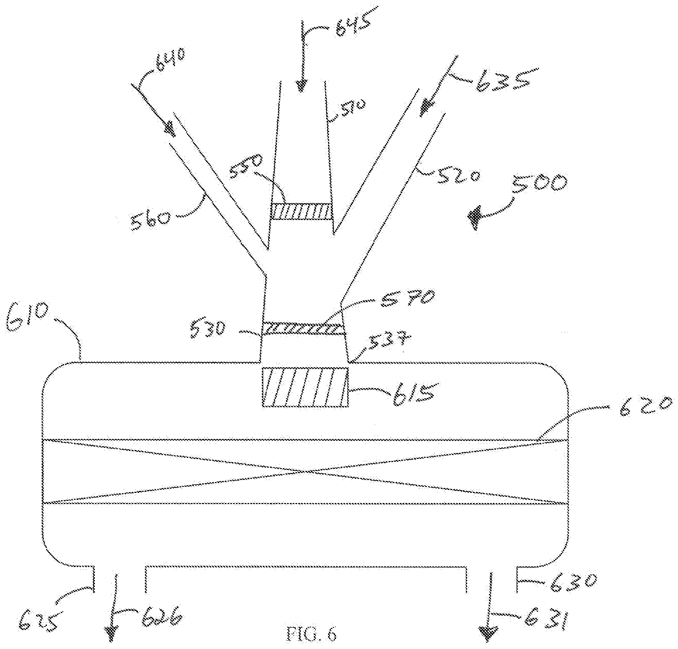

Figure 6 depicts a cross-sectional elevational view of an illustrative reactor system 600 for dehydrogenating one or more alkanes that includes the inlet assembly 500 discussed and described above with reference to Figure 5 coupled to a dehydrogenation reactor 610, according to one or more embodiments. The reactor 610 can include one or more gas distributors 615 and one or more catalyst beds 620. In some examples, the catalyst bed 620 can be a fixed catalyst bed. The reactor can also include an outlet 625 for recovering a crude dehydrogenated product via line 626 therefrom. The reactor 610 can also include one or more purge gas or off-gas outlets 630 for recovering one or more purge gases, off-gases, regeneration gases, or other fluids via line 631, *e.g.,* fluids introduced and/or produced during a catalyst decoking and regeneration process. Suitable dehydrogenation process conditions, feeds, and other parameters can be or include those discussed and described in U.S. Patent Nos.: 2,423,029, 4,560,824, and 4,581,339; and WO Publication No. WO 2016/069918.

One or more alkanes via line 635 can be introduced to the second conduit 520 of the inlet assembly 500. The alkane can pass through inlet assembly 500 and into the reactor 610. As the alkane passes through the catalyst bed 620, the alkane can contact the catalyst to produce a crude product that can include one or more alkenes, such as propylene or butylene, and a coked catalyst that can include coke disposed on the catalyst. The dehydrogenation of the alkane is an endothermic reaction. Accordingly, the alkane and/or the catalyst can be heated to a temperature sufficient to provide at least a portion of the heat required to dehydrogenate the alkane in the presence of the catalyst. The catalyst can be a fresh or new catalyst, a regenerated catalyst, or a mixture thereof.

Illustrative alkanes can be or include, but are not limited to, ethane, propane, butane, 2-methylpropane, pentane, 2-methylbutane, 2,2-dimethylpropane, isomers thereof, or any mixture thereof. The dehydrogenated product can be or include, but is not limited to, ethene (ethylene), propene (propylene), but-1-ene (butylene), (*Z*)-but-2-ene, (*E*)-but-2-ene, 2-methylpropene, 1-pentene, *cis*-2-pentene, *trans*-2-pentene, isomers thereof, or any mixture thereof. In at least one example, the alkane can be or include propane and the dehydrogenated product can be or include propylene. For example, the alkane can include at least 80 wt% of propane, at least 85 wt% of propane, at least 90 wt% of propane, at least 95 wt% of propane, at least 97 wt% of propane, at least 98 wt% of propane, at least 99 wt% of propane, at least 99.5 wt% of propane, at least 99.7 wt% of propane, or at least 99.9 wt% of propane. In other examples, the alkane can include at least 80 wt% of butane, at least 85 wt% of butane, at least 90 wt% of butane, at least 95 wt% of butane, at least 97 wt% of butane, at least 98 wt% of butane, at least 99 wt% of butane, at least 99.5 wt% of butane, at least 99.7 wt% of butane, or at least 99.9 wt% of butane. The dehydrogenation of the alkane to the alkene can be incomplete. As such, the crude dehydrogenated product can include the alkene and the alkane that was not dehydrogenated. In one or more examples, at least a portion of the alkane present in the crude dehydrogenated product can be separated therefrom to produce a purified dehydrogenated product. The alkane separated from the crude dehydrogenated product can be recycled or combined with fresh or incoming alkane and contacted with the catalyst.

The catalyst can be at a temperature of about 425°C, about 450°C, about 475°C, about 500°C, about 535°C, about 550°C, about 565°C, about 585°C or about 590°C to about 595°C, about 600°C, about 625°C, about 650°C, about 675°C, about 700°C, about 750°C, or about 800°C when contacted with the alkane. The catalyst can include one or more metals. The one or more metals can include, but are not limited to, platinum, palladium, gold, indium, chromium, tin, iron, molybdenum, tungsten, nickel, silver, any alloy thereof, any oxide thereof, or any mixture thereof. The one or more metals can be supported or unsupported. Illustrative supports can include, but are not limited to, one or more inorganic oxides. Illustrative inorganic oxides can include, but are not limited to, alumina, silica, titania, zirconia, boria, zinc oxide, magnesia, or any mixture thereof. Illustrative combinations of inorganic oxides can include, but are not limited to, alumina-silica, silica-titania, alumina-silica-titania, alumina-zirconia, alumina-titania, or any mixture thereof. In at least one example, the support can be or include alumina, silica, or a combination thereof. The catalyst can be disposed within the reactor within a fixed bed and the alkane can be introduced to a first side of the fixed bed and the dehydrogenated product can be recovered from a second side of the fixed bed. In at least one example, the first side and the second side of the fixed bed can be on opposite sides of the fixed bed with respect to one another.

When the amount of coke deposited on, in, or otherwise about the catalyst and/or the temperature of the catalyst reduces such that the reaction rate and/or conversion rate of the alkane to the alkene falls below a desired level, the alkane can be diverted to another reactor and the coked catalyst can be contacted with a purge fluid introduced via line 640 to the third conduit 560 to remove at least a portion of any residual alkane on, about, and/or within the catalyst bed 620 and/or within the reactor 610. The purge fluid can be or include, but is not limited to, nitrogen, argon, helium, carbon dioxide, exhaust or combustion gas, air, water or steam, or any mixture thereof. In at least one example, the purge fluid can be or include steam. The purge fluid, *e.g*., steam, can be at a temperature of about 120°C, about 150°C, about 175°C, about 200°C, about 225°C, about 250°C, or about 275°C to about 400°C, about 450°C, about 500°C, about 525°C, about 550°C, about 575°C, or about 600°C. For example, the purge fluid, *e.g*., steam, can be at a pressure of about 101 kPa, about 125 kPa, about 200 kPa, about 350 kPa, about 500 kPa, about 1,000 kPa, about 1,500 kPa, about 2,000 kPa, or about 2,500 kPa to about 3,000 kPa, about 3,500 kPa, about 4,000 kPa, or about 4,500 kPa.

After contacting the catalyst with the purge fluid, the catalyst can be decoked. Decoking the coked catalyst can remove at least a portion of the coke deposited on, in, and/or about the catalyst. The coke can be removed from the catalyst by combustion of the coke. For example, the catalyst can be contacted with an oxygen-containing fluid at conditions sufficient to combust at least a portion of the coke, thereby removing coke from the coked catalyst. Illustrative oxygen-containing fluids can be or include, but are not limited to, ambient air, bottled air, in-line or in-house air, oxygen (O₂), nitrous oxide, ozone, or any mixture thereof. In at least one example, the oxygen-containing fluid can be or include air. The oxygen-containing fluid can be introduced via line 645 to the first conduit 510. The oxygen-containing fluid can flow through the inlet assembly 500 and into the reactor 610.

The oxygen-containing fluid can be at a temperature of about 500°C, about 550°C, about 600°C, about 650°C, about 660°C, about 665°C, about 670°C, or about 675°C to a temperature of about 680°C, about 685°C, about 690°C, about 695°C, about 700°C, about 725°C, about 750°C, about 775°C, or about 800°C when flowing through the inlet assembly 500 and/or when contacted with the coked catalyst to produce the regenerated catalyst. The oxygen-containing fluid can be at a pressure of about 101 kPa, about 250 kPa, about 500 kPa, or about 750 kPa to about 1,000 kPa, about 1,250 kPa, about 1,500 kPa, about 1,750 kPa, about 2,500 kPa, about 3,000 kPa, about 3,500 kPa, about 4,000 kPa, or about 4,500 kPa when flowing through the inlet assembly 500 and/or when contacted with the coked catalyst to produce the regenerated catalyst. In some examples, the oxygen-containing fluid can be at a pressure of about 101 kPa, about 110 kPa, or about 115 kPa to about 125 kPa, about 135 kPa, or about 150 kPa when flowing through the inlet assembly 500 and/or when contacted with the coked catalyst to produce the regenerated catalyst.

In some example, the oxygen-containing fluid can flow through the inlet assembly 500 at a velocity of about 100 m/s, about 125 m/s, about 150 m/s, about 175 m/s, or about 190 m/s to about 210 m/s, about 225 m/s, or about 250 m/s. The amount of the oxygen-containing fluid that can be introduced to the reactor 610 and contacted with the coke catalyst to produce the decoked catalyst can widely vary. For example, the amount of the oxygen-containing fluid introduced to the reactor 610 and contacted with the coked catalyst to produce the decoked catalyst can be about 300 m³/s, about 325 m³/s, or about 350 m³/s to about 375 m³/s, about 400 m³/s, or about 425 m³/s.

The oxygen-containing fluid can have a velocity profile at the second end 537 of the outlet conduit 530. The second end 537 of the outlet conduit 530 can have a radial distance from the inner surface 534 thereof to the central longitudinal axis 532 thereof. The velocity maxima of the velocity profile at the second end 537 of the outlet conduit 530 can be at least 80% of the radial distance. In some examples, the velocity maxima of the velocity profile at the second end 537 of the outlet conduit 530 can be at least 82%, at least 84%, at least 86%, at least 88%, at least 90%, at least 92%, at least 93%, at least 94%, at least 94.5%, at least 95%, at least 95.5%, at least 96%, at least 96.5%, at least 97%, at least 97.5%, or more of the radial distance. In some examples, the velocity maxima of the velocity profile of the oxygen-containing fluid at the second end 537 of the outlet conduit 530 can be at least 80%, at least 82%, at least 83%, at least 84%, at least 85%, at least 86%, at least 87%, at least 88%, at least 89%, at least 90%, at least 91%, at least 92%, at least 93%, at least 94%, at least 94.5%, at least 95%, at least 95.5%, at least 96%, at least 96.5%, at least 97%, at least 97.5%, or more of a radial distance away from the inner surface 534 thereof when a velocity of the oxygen-containing fluid is greater than or equal to 100 m/s, 125 m/s, 150 m/s, 175 m/s, and/or 200 m/s. As noted above, the velocity maxim of the velocity profile can be measured, computed, determined, or otherwise estimated via computational fluid dynamics (CFD) modeling. For example, the CFD modeling can use a CFD solver and a meshing solver. In some examples the CFD solver can be the Ansys CFX V17.2 and the meshing solver can be the Ansys Mesher V17.2. The oxygen-containing fluid introduced via line 645 to the first conduit 510 can flow through the inlet assembly 500 in the turbulent flow regime.

In some examples, one or more hydrocarbon fuels can be contacted with the coked catalyst while the oxygen-containing fluid contacts the coked catalyst. The hydrocarbon fuel can combust in the presence of the oxygen-containing fluid to increase the temperature of the decoked catalyst. For example, the hydrocarbon fuel can be introduced via line 640 to the third conduit 560 and the oxygen-containing fluid can be introduced via line 645 to the first conduit 510. For example, contact between the oxygen-containing fluid and the coked catalyst can be started, then contact between the hydrocarbon fuel and the coked catalyst can be started, then contact between the hydrocarbon fuel can be stopped, and then contact between the oxygen-containing fluid can be stopped. In another example, contact between the oxygen-containing fluid and the hydrocarbon fuel can be started at the same or substantially the same time, *e.g.,* within 10 seconds of one another, then contact between the hydrocarbon fuel can be stopped, and then contact between the oxygen-containing fluid can be stopped.

The hydrocarbon fuel can be a gas and/or a liquid hydrocarbon. Illustrative hydrocarbon fuels can include, but are not limited to, one or more C₁-C₆ hydrocarbons such as methane, ethane, propane, butane, pentane, hexane, or any mixture thereof. In at least one example, the hydrocarbon fuel can be or include methane and can be contacted with the coked catalyst while the oxygen-containing fluid contacts the coked catalyst. In another example, the hydrocarbon fuel can include at least 90 wt%, at least 93 wt%, at least 95 wt%, at least 97 wt%, or at least 99 wt% methane. In at least one other example, the hydrocarbon fuel can be or include natural gas.

The decoked catalyst can be contacted with one or more reducing gases to produce a regenerated catalyst and an off-gas. For example, the reducing gas can be introduced via line 640 to the third conduit 560 and into the reactor 610 via the inlet assembly 500. The reducing gas can contact the decoked catalyst therein to produce the regenerated catalyst and the off-gas. The reducing gas can remove at least a portion of any adsorbed oxygen from the decoked catalyst. For example, if the catalyst includes chromium, the reducing gas can convert at least a portion of any chromium (VI) to chromium (III). The off-gas can include at least a portion of the adsorbed oxygen that can be removed from the decoked catalyst. The off-gas, oxygen-containing fluid, combustion gas products, reducing gas, and purge gas can be removed from the reactor 620 via line 631 from outlet 630. Once the coked catalyst has been decoked and regenerated additional alkane can be introduced to the reactor 610 to produce additional dehydrogenated products.

Illustrative reducing gases can be or include, but are not limited to, hydrogen, methane, ethane, ethylene, propane, propylene, carbon monoxide, butane, or any mixture thereof. For example, the reducing gas can be or include a hydrogen product recovered from one or more pressure swing absorption units that separates a gas mixture containing hydrogen and one or more other components such as methane, ethane, carbon dioxide, nitrogen, oxygen, ammonia, or any mixture thereof. In another example, the reducing gas can be or include hydrogen recovered via membrane separation of a gas mixture containing hydrogen and one or more other components such as methane, ethane, carbon dioxide, nitrogen, oxygen, ammonia, or any mixture thereof.

### Prophetic Examples

In order to provide a better understanding of the foregoing discussion, the following non-limiting prophetic examples are offered. Although the simulated examples are directed to specific embodiments, they are not to be viewed as limiting the invention in any specific respect.

Computational Fluid Dynamics (CFD) modeling is used to simulate the velocity contour profile of air flowing through large and a small sized models of an inlet assembly for introducing a fluid into a reactor. The CFD solver is Ansys CFX V17.2 and the meshing solver is Ansys Mesher V17.2. The mesh details for geometry with no internals based on inlet diameter being equal to D are as follows: mesh type is fully tetrahedral with wall inflation; size function refinement is proximity and curvature; number of elements is greater than 1800000, number of wall inflation layers is 7, maximum face size is about 0.0800D; minimum face sizing is about 0.0123D; and maximum tetrahedral size is about 0.0984D.

The simulations assume the air is a single phase gas, viscous, compressible, and turbulent (K-Qsst). The flow conditions of the air flowing through the large and small sized inlet assemblies are shown in Table 1 below.

| Table 1: Flow Conditions of the Air | | |
|---|---|---|
| Model | Large Model | Small Model |
| Air Volume Flow Rate (ft³/s) | 12,000 | 3,400 |
| Temperature (°C) | 650 | 650 |
| Pressure (atm) | 1.2 | 1.2 |
| Velocity of air (m/s) | 200 | 200 |

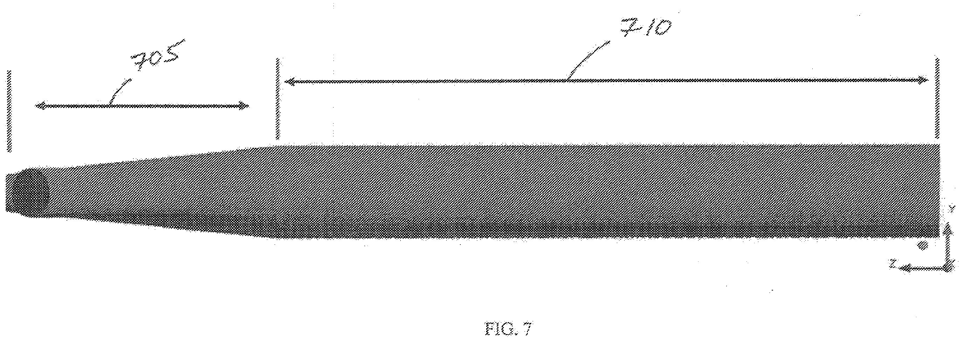

Figure 7 depicts a CFD inlet assembly model that is used in the CFD modeling of both large and small sized inlet assemblies in Examples C1, C2, and Ex. 1-4. The CFD model includes a viscous section 705 and an inviscid section 710. The inviscid section 710 is added for numerical stability only.

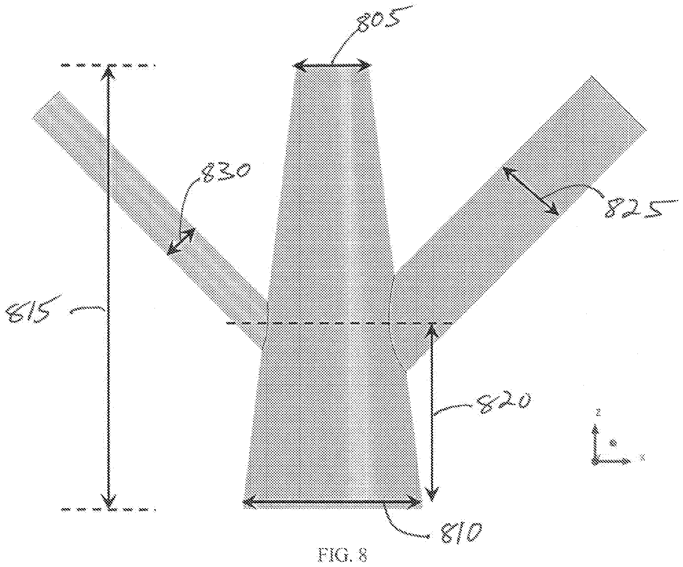

Table 2 lists the relevant geometric dimensions of the large and small inlet assemblies that are used in the simulations. The longitudinal axis of the first inlet conduit and the longitudinal axis of the outlet conduit are axially aligned with one another. The dimensions listed in Table 2 are indicated in Figure 8.

| Table 2: Dimensions | | |
|---|---|---|
| Model | Large Model | Small Model |
| First Inlet Conduit Diameter (805) | 0.403 x D_{L} | 0.405 x D_{S} |
| Outlet Conduit Diameter (810) | D_{L} | Ds |
| Length of Inlet assembly (815) | 2.416 x D_{L} | 2.643 x Ds |
| Length from Outlet End to Center of Junction (820) | 0.966 x D_{L} | 1.063 x D_{S} |
| Second Inlet Conduit Diameter (825) | 0.430 x D_{L} | 0.532 x D_{S} |
| Third Conduit Diameter (830) | 0.215 x D_{L} | 0.228 x D_{S} |

### Large Inlet Assembly CFD Simulations

Three large inlet assembly CFD simulations are conducted. The first simulation (C1) is a comparative example that does not include any pre-distributor. The second and third simulations (Ex. 1 and Ex. 2) are inventive examples that include a pre-distributor. Ex. 1 includes five rings, each having a constant width. Three rings are disposed on an inner surface of the first conduit about an inner perimeter thereof and two rings are disposed on an inner surface of the outlet conduit about an inner perimeter thereof. Ex. 2 includes three lobed rings, *i.e.,* rings having semi-elliptical tabs disposed about an inner surface thereof. One lobed ring is disposed on an inner surface of the first conduit about an inner perimeter thereof and two lobed rings are disposed on an inner surface of the outlet conduit about an inner perimeter thereof.

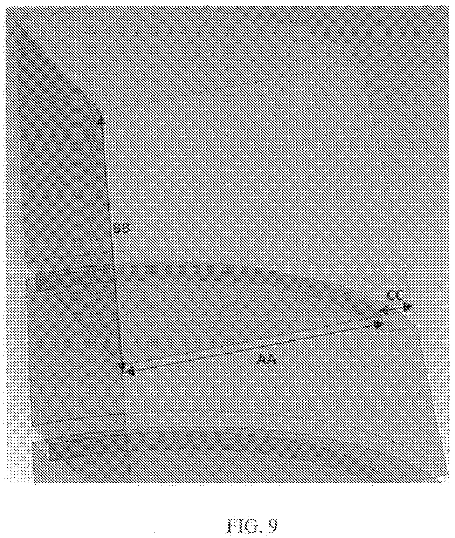

Table 3 lists the distance from the longitudinal axis of the first inlet conduit or the outlet conduit (depending on the particular ring) to the inner surface of each ring disposed therein (AA), the distance each ring is from the end of the first inlet conduit (BB), and the width of each ring (CC). Figure 9 illustrates the distances AA, BB, and CC provided in Table 3.

| Table 3: Dimensions for Large Model with 5 Rings | | | | |
|---|---|---|---|---|
| Ring Position | Ring Thickness | Distance from longitudinal axis to inner surface of ring (AA) | Distance from end of first inlet conduit to ring (BB) | Width of ring (CC) |
| 1 | 0.01321 x D_{L} | 0.45326 x D_{L} | 0.20476 x D_{L} | 0.02531 x D_{L} |
| 2 | 0.01321 x D_{L} | 0.48711 x D_{L} | 0.33686 x D_{L} | 0.04164 x D_{L} |
| 3 | 0.01321 x D_{L} | 0.51857 x D_{L} | 0.46896 x D_{L} | 0.05797 x D_{L} |
| 4 | 0.01321 x D_{L} | 0.91048 x D_{L} | 2.05416 x D_{L} | 0.05894 x D_{L} |
| 5 | 0.01321 x D_{L} | 0.0.94313 x D_{L} | 2.18626 x D_{L} | 0.07527 x D_{L} |

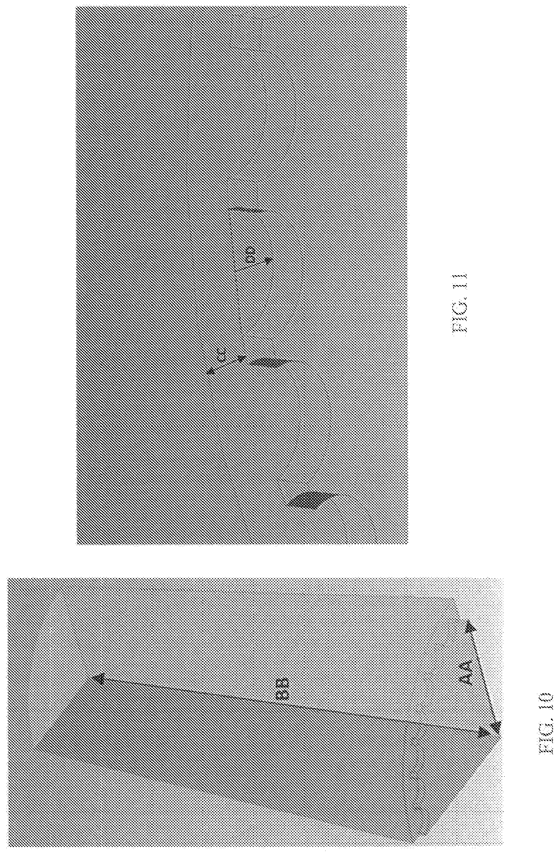

Table 4 lists the distance from the longitudinal axis of the first inlet conduit or the outlet conduit (depending on the particular ring) to the inner surface of each lobed ring at a position corresponding to the center of each lobe (AA), the distance each ring is from the end of the first inlet conduit (BB), the width of each ring (CC), and the radius of each lobe (DD). Figures 10 and 11 illustrate the distances AA, BB, CC, and DD provided in Table 4. Each lobed ring has 30 lobes, *i.e.,* semi-elliptical tabs disposed about an inner surface of the ring.

| Table 4: Dimensions for Large Model with 3 lobed Rings | | | | | |
|---|---|---|---|---|---|
| Ring Position | Ring Thickness | Distance from longitudinal axis to inner surface of ring at center of lobe (AA) | Distance from end of first inlet conduit to ring (BB) | Width of ring (CC) | Radius of lobe (DD) |
| 1 | 0.01321 x D_{L} | 0.60637 x D_{L} | 0.82404 x D_{L} | 0.02396 x D_{L} | 0.02351 x D_{L} |
| 2 | 0.01321 x D_{L} | 0.86110 x D_{L} | 1.85443 x D_{L} | 0.03348 x D_{L} | 0.03322 x D_{L} |
| 3 | 0.01321 x D_{L} | 0.91336 x D_{L} | 2.06579 x D_{L} | 0.03543 x D_{L} | 0.03527 x D_{L} |

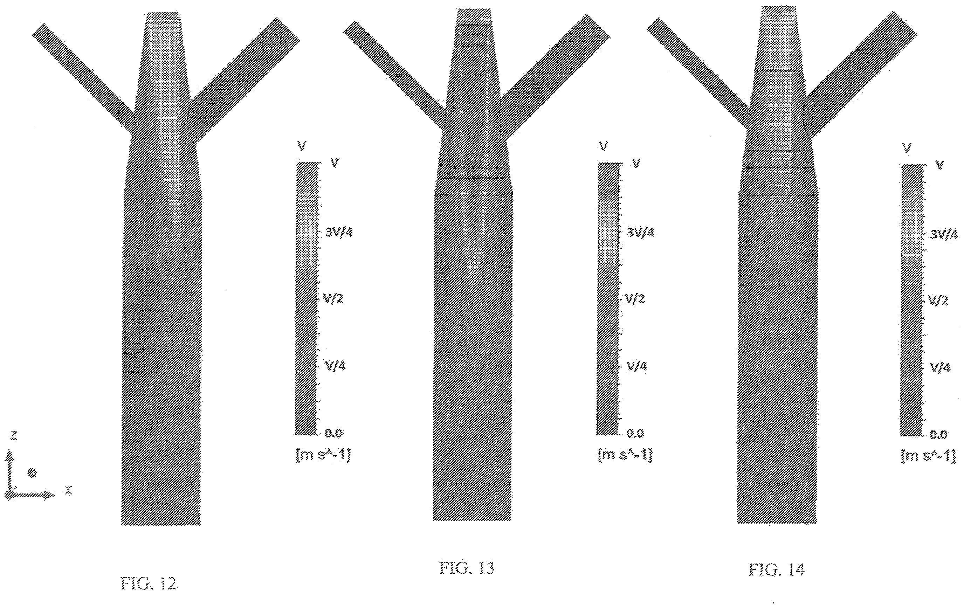

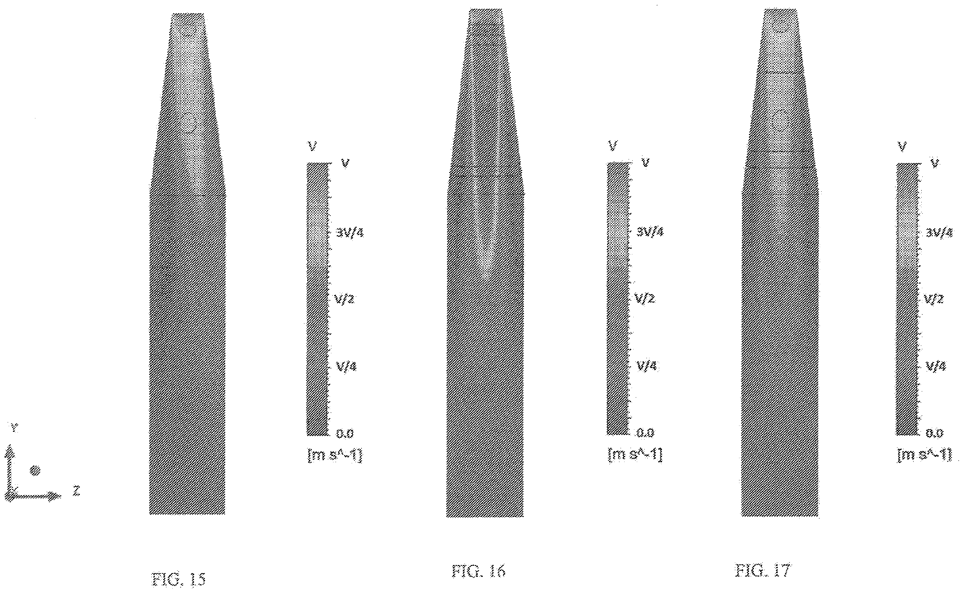

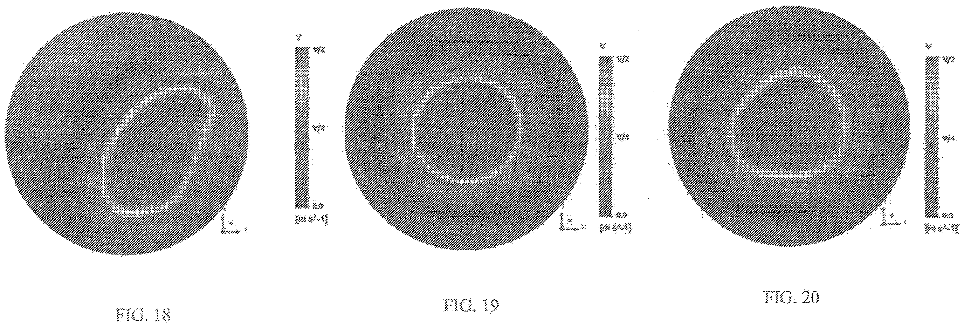

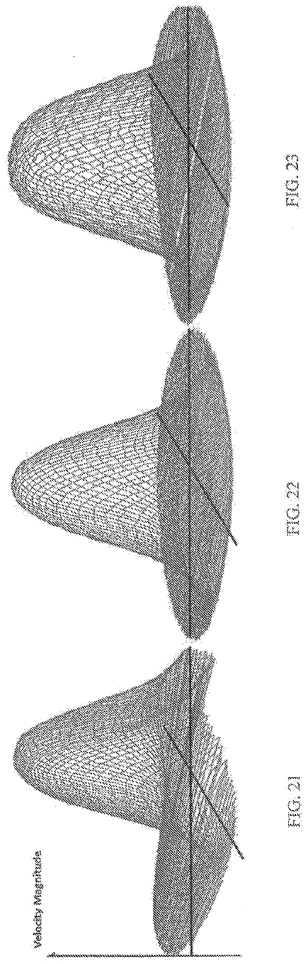

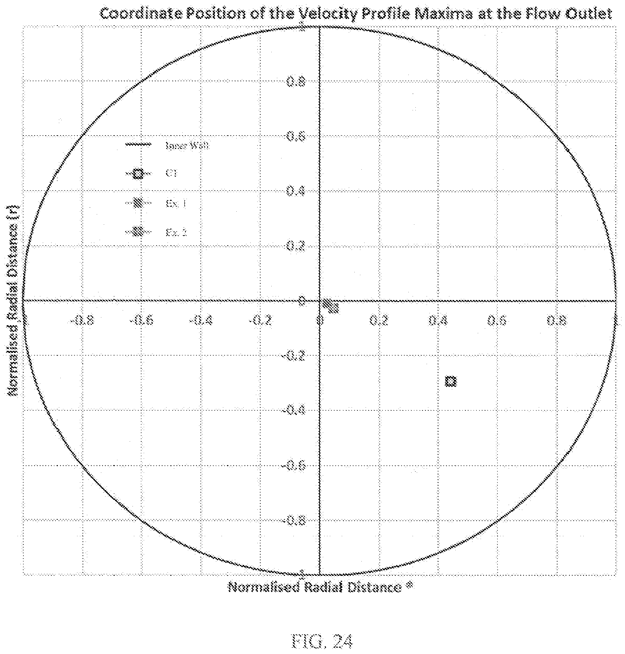

Figures 12, 13, and 14 depict the simulated air velocity contours through the inlet assemblies along the ZX-plane in examples C1, Ex. 1, and Ex. 2, respectively. Figures 15, 16, and 17 depict the simulated air velocity contours through the inlet assemblies along the YZ-plane in examples C1, Ex. 1, and Ex. 2, respectively. Figures 18, 19, and 20 depict the simulated air velocity fields at the end of the outlet conduit of the inlet assemblies in examples C1, Ex. 1, and Ex. 2, respectively. Figures 21, 22, and 23 depict the approximate 3D-structure of the simulated velocity profiles at the end of the outlet conduit of the inlet assemblies in Examples C1, Ex. 1, and Ex. 2, respectively. Figure 24 depicts a coordinate position of the simulated velocity profile maxima at the end of the outlet conduit of the inlet assemblies in Examples C1, Ex. 1, and Ex. 2, respectively

As can be seen from Figures 12-24, the velocity centroids for the two inventive examples (Ex. 1 and Ex. 2) are significantly more centered than the comparative example (C1) that does not include a pre-distributor. In fact, the positional accuracy of the velocity profile maxima, *i.e.,* the percent of radial position where the velocity maxima is found, for Ex. 1 and Ex. 2 are 97.6% and 94.5%, respectively. In contrast, the positional accuracy of the velocity profile maxima for comparative example (C1) is only 47.2%. The pressure loss for the comparative example (C1) and inventive examples (Ex. 1 and Ex. 2) is 2,349.7 Pa, 7,638.2 Pa, and 2,510.2 Pa, respectively.

### Small Inlet Assembly CFD Simulations

Three small inlet assembly CFD simulations are conducted. The first simulation (C2) is a comparative example that does not include any pre-distributor. The second and third simulations (Ex. 3 and Ex. 4) are inventive examples that include a pre-distributor. Ex. 3 includes four rings, each having a constant width, as the pre-distributors. Two rings are disposed on an inner surface of the first conduit about an inner perimeter thereof and two rings are disposed on an inner surface of the outlet conduit about an inner perimeter thereof. Ex. 2 includes three lobed rings, *i.e.,* rings having semi-elliptical tabs disposed about an inner surface thereof. One lobed ring is disposed on an inner surface of the first conduit about an inner perimeter thereof and two lobed rings are disposed on an inner surface of the outlet conduit about an inner perimeter thereof.

Table 5 lists the distance from the longitudinal axis of the first inlet conduit or the outlet conduit (depending on the particular ring) to the inner surface of each ring disposed therein (AA), the distance each ring is from the end of the first inlet conduit (BB), and the width of each ring (CC). The distances AA, BB, and CC provided in Table 5 are the same as those in the large simulations and are shown in Figure 9.

| Table 5: Dimensions for Small Model with 4 Rings | | | | |
|---|---|---|---|---|
| Ring Position | Ring Thickness | Distance from longitudinal axis to inner surface of ring (AA) | Distance from end of first inlet conduit to ring (BB) | Width of ring (CC) |
| 1 | 0.02491 x D_{S} | 0.46426 x D_{S} | 0.26334 x D_{S} | 0.02964 x D_{S} |
| 2 | 0.02491 x D_{S} | 0.50912 x D_{S} | 0.46264 x D_{S} | 0.05207 x D_{S} |
| 3 | 0.02491 x D_{S} | 0.91289 x D_{S} | 2.25635 x D_{S} | 0.03292 x D_{S} |
| 4 | 0.02491 x D_{S} | 0.95775 x D_{S} | 2.45566 x D_{S} | 0.05535 x D_{S} |

Table 6 lists the distance from the longitudinal axis of the first inlet conduit or the outlet conduit (depending on the particular ring) to the inner surface of each lobed ring at a position corresponding to the center of each lobe (AA), the distance each ring is from the end of the first inlet conduit (BB), the width of each ring (CC), and the radius of each lobe (DD). The distances AA, BB, CC, and DD provided in Table 6 are the same distances referred to with regard to the large model simulations and are also indicated in Figures 10 and 11. Each lobed ring has 30 lobes, *i.e.,* semi-elliptical tabs disposed about an inner surface of the ring.

| Table 6: Dimensions for Small Model with 3 Rings | | | | | |
|---|---|---|---|---|---|
| Ring Position | Ring Thickness | Distance from longitudinal axis to inner surface of ring at center of lobe (AA) | Distance from end of first inlet conduit to ring (BB) | Width of ring (CC) | Radius of lobe (DD) |
| 1 | 0.01321 x D_{S} | 0.61006 x D_{S} | 0.91106 x D_{S} | 0.02838 x D_{S} | 0.02292 x D_{S} |
| 2 | 0.01321 x D_{S} | 0.86801 x D_{S} | 2.05705 x D_{S} | 0.04284 x D_{S} | 0.03413 x D_{S} |
| 3 | 0.01321 x D_{S} | 0.92409 x D_{S} | 2.30618 x D_{S} | 0.04598 x D_{S} | 0.03687 x D_{S} |

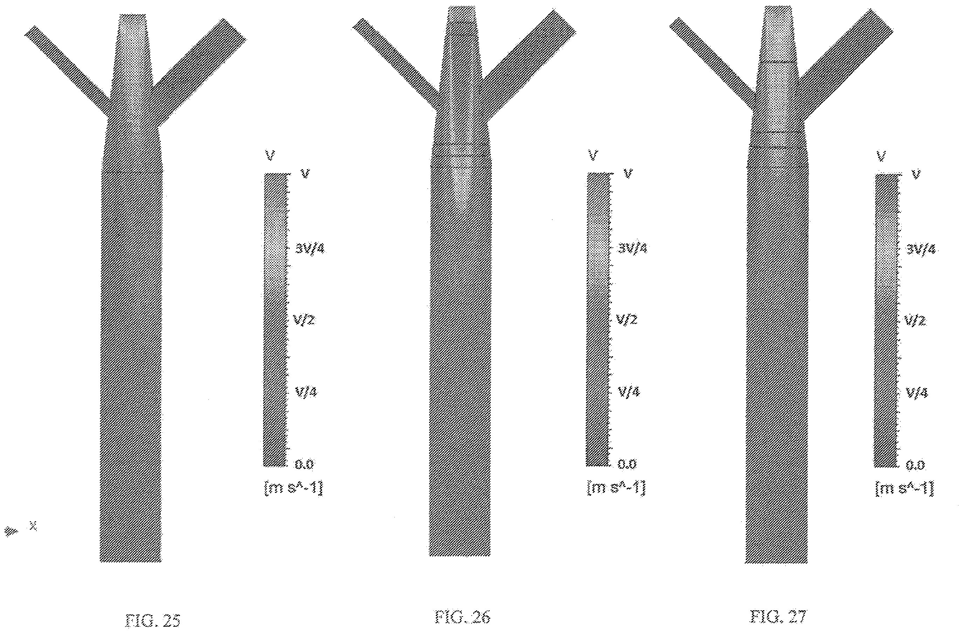

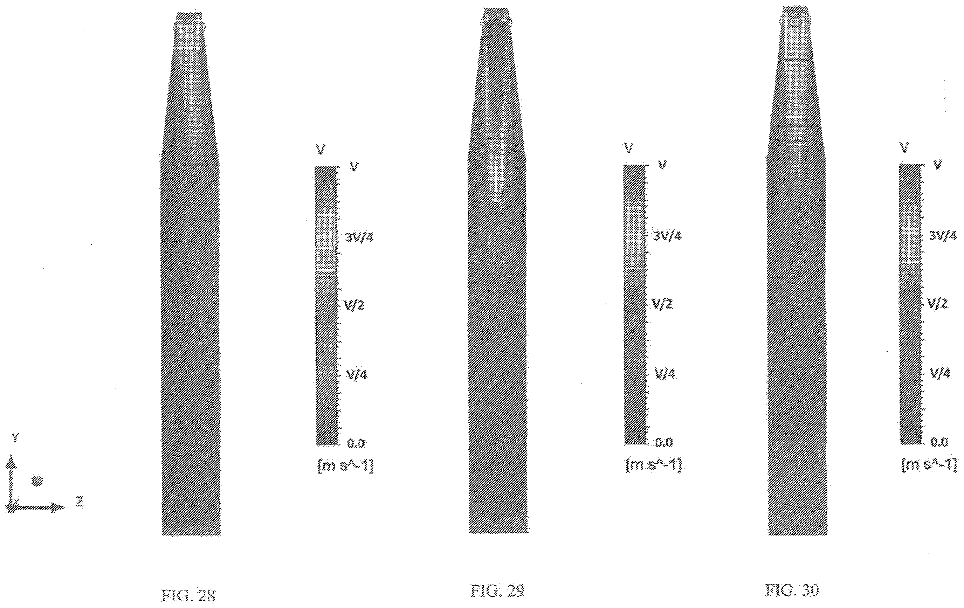

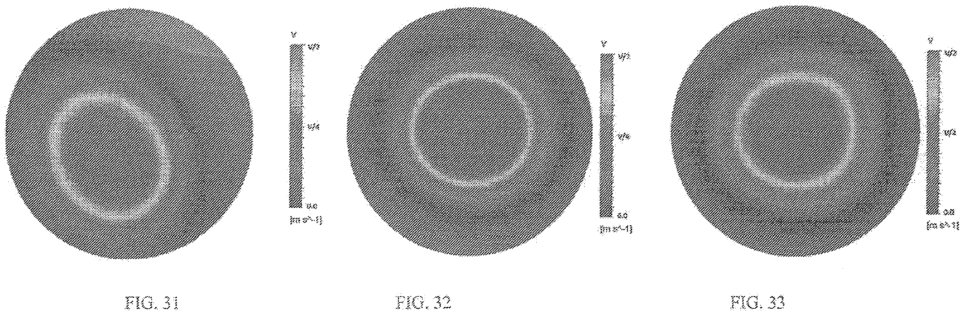

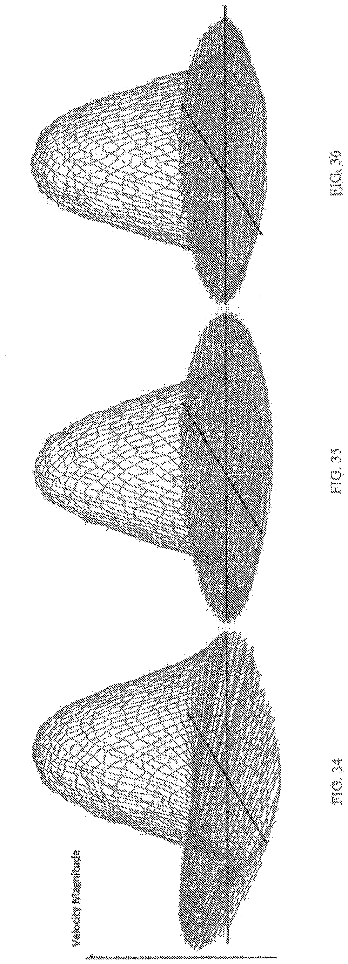

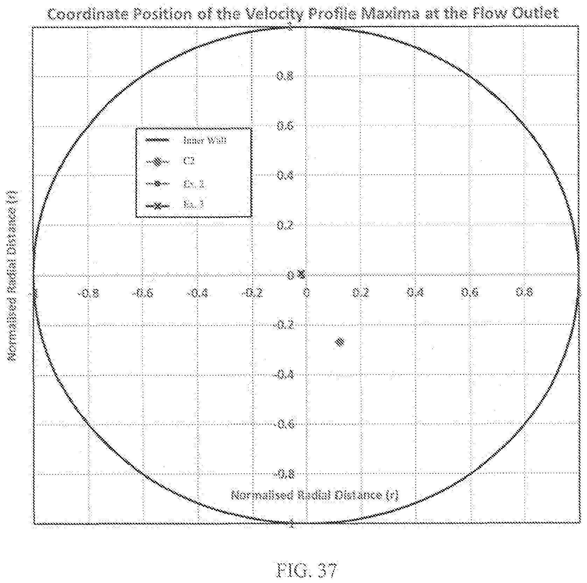

Figures 25, 26, and 27 depict the simulated air velocity contours through the inlet assemblies along the ZX-plane in examples C2, Ex. 3, and Ex. 4, respectively. Figures 28, 29, and 30 depict the simulated air velocity contours through the inlet assemblies along the YZ-plane in examples C2, Ex. 3, and Ex. 4, respectively. Figures 31, 32, and 33 depict the simulated air velocity fields at the end of the outlet conduits of the inlet assemblies in examples C2, Ex. 3, and Ex. 4, respectively. Figures 34, 35, and 36 depict an approximate 3D-structure of the simulated velocity profiles at the end of the outlet conduits of the inlet assemblies in Examples C2, Ex. 3, and Ex. 4, respectively. Figure 37 depicts a coordinate position of the simulated velocity profile maxima at the end of the outlet conduits of the inlet assemblies of Examples C2, Ex. 3, and Ex. 4, respectively.

As can be seen from Figures 20-37, the velocity centroids of the two inventive examples (Ex. 3 and Ex. 4) are significantly more centered than the comparative example (C2) that does not include a pre-distributor. In fact, the positional accuracy of the velocity profile maxima, *i.e.,* the percent of radial position where the velocity maxima is found, for Ex. 3 and Ex. 4 are 98.6% and 97.9%, respectively. In contrast, the positional accuracy of the velocity profile maxima for comparative example (C2) is only 70.6%. The pressure loss for the comparative example (C2) and inventive examples (Ex. 3 and Ex. 4) is 2,204.4 Pa, 7,769.4 Pa, and 2,796.0 Pa, respectively.

Embodiments of the present disclosure further relate to any one or more of the following paragraphs:
1. A process for dehydrogenating an alkane, comprising: introducing an alkane into a reactor from an outlet conduit of an inlet assembly; contacting a catalyst within the reactor with the alkane to produce a dehydrogenated product comprising an alkene and a coked catalyst comprising coke disposed on the catalyst; separating the dehydrogenated product from the coked catalyst; introducing an oxygen-containing fluid into the reactor from the outlet conduit, wherein the oxygen-containing fluid has a velocity profile at an end of the outlet conduit coupled to the reactor, wherein the end of the outlet conduit coupled to the reactor has a radial distance from an inner surface thereof to a center thereof, and wherein a velocity maxima of the velocity profile at the end of the outlet conduit coupled to the reactor is at least 80% of the radial distance away from the inner surface thereof when a velocity of the oxygen-containing fluid is greater than or equal to 100 m/s; and combusting at least a portion of the coke disposed on the catalyst in the presence of the oxygen-containing fluid to produce a decoked catalyst.
2. A process for dehydrogenating an alkane, comprising: introducing an alkane into a reactor from an outlet conduit of an inlet assembly; contacting a catalyst within the reactor with the alkane to produce a dehydrogenated product comprising an alkene and a coked catalyst comprising coke disposed on the catalyst; separating the dehydrogenated product from the coked catalyst; introducing an oxygen-containing fluid into the reactor from the outlet conduit, wherein the oxygen-containing fluid has a velocity profile at an end of the outlet conduit coupled to the reactor, wherein the end of the outlet conduit coupled to the reactor has a radial distance from an inner surface thereof to a center thereof, and wherein a velocity maxima of the velocity profile at the end of the outlet conduit coupled to the reactor is at least 80% of the radial distance away from the inner surface thereof when a velocity of the oxygen-containing fluid is greater than or equal to 100 m/s, as determined using computational fluid dynamics modeling; and combusting at least a portion of the coke disposed on the catalyst in the presence of the oxygen-containing fluid to produce a decoked catalyst.
3. The process according to paragraphs 1 or 2, further comprising: contacting the decoked catalyst with a reducing gas to produce a regenerated catalyst and an off-gas; and contacting the regenerated catalyst with additional alkane to produce additional dehydrogenated product and additional coked catalyst.
4. The process according to any one of paragraphs 1 to 3, wherein: the oxygen-containing fluid is introduced through a first inlet conduit coupled to a junction of the inlet assembly, the alkane is introduced through a second inlet conduit coupled to the junction of the inlet assembly, the outlet conduit is coupled to the junction, there is an acute angle between a longitudinal axis of the second inlet conduit and a longitudinal axis of the first inlet conduit, and wherein there is an obtuse angle between the longitudinal axis of the second inlet conduit and a longitudinal axis of the outlet conduit.
5. The process of paragraph 4, wherein the oxygen-containing fluid has a velocity profile at an end of the outlet conduit coupled to the junction, wherein the end of the outlet conduit coupled to the junction has a radial distance from an inner surface thereof to a center thereof, and wherein a velocity maxima of the velocity profile at the end of the outlet conduit coupled to the junction is at least 80% of the radial distance away from the inner surface thereof when a velocity of the oxygen-containing fluid is greater than or equal to 100 m/s.
6. The process of paragraph 5, wherein the radial distance at the end of the outlet conduit coupled to the junction is less than the radial distance at the end of the outlet conduit coupled to the reactor.
7. The process according to any one of paragraphs 4 to 6, wherein the outlet conduit has a frusto-conical inner surface.
8. The process according to any one of paragraphs 4 to 7, wherein the first inlet conduit comprises a pre-distributor disposed therein.
9. The process of paragraph 8, wherein the pre-distributor comprises a band of material disposed on an inner surface of the first inlet conduit.
10. The process of paragraph 9, wherein the band of material has a substantially symmetrical inner surface.
11. The process of paragraph 9, wherein the band of material comprises a ring having a substantially constant width.
12. The process of paragraph 9, wherein the band of material comprises a ring having a plurality of tabs attached to an inner surface thereof, wherein the plurality of tabs is positioned to provide the pre-distributor with a substantially symmetrical inner surface.
13. The process of paragraph 12, wherein each tab has a geometrical shape selected from the group consisting of: a semi-rectangle and a semi-ellipse.
14. The process of paragraph 8, wherein the pre-distributor comprises a plurality of discrete tabs disposed on an inner surface of the first inlet conduit, wherein a location of each discrete tab is substantially the same along the longitudinal axis of the first inlet conduit with respect to one another.
15. The process of paragraph 14, wherein the pre-distributor has a substantially symmetrical inner surface.
16. The process of paragraph 8, wherein the pre-distributor comprises a plurality of rings disposed on an inner surface of the first inlet conduit.
17. The process of paragraph 8, wherein the pre-distributor comprises two bands of material disposed on an inner surface of the first inlet conduit, and wherein the two bands of material are spaced apart from one another along the longitudinal axis of the first inlet conduit.
18. The process of paragraph 8, wherein the pre-distributor comprises three bands of material disposed on an inner surface of the first inlet conduit, and wherein the two bands of material are spaced apart from one another along the longitudinal axis of the first inlet conduit.
19. The process of paragraph 8, wherein the pre-distributor comprises 2, 3, 4, 5, 6, 7, 8, 9, or 10 rings disposed on an inner surface of the first inlet conduit.
20. The process of paragraph 8, wherein the pre-distributor is disposed on an inner surface of the first inlet conduit.
21. The process according to any one of paragraphs 1 to 20, wherein the velocity maxima of the velocity profile at the end of the outlet conduit coupled to the reactor is at least 83% of the radial distance away from the inner surface thereof when the velocity of the oxygen-containing fluid is greater than or equal to 100 m/s.
22. The process according to any one of paragraphs 1 to 20, wherein the velocity maxima of the velocity profile at the end of the outlet conduit coupled to the reactor is at least 85% of the radial distance away from the inner surface thereof when the velocity of the oxygen-containing fluid is greater than or equal to 100 m/s.
23. The process according to any one of paragraphs 1 to 20, wherein the velocity maxima of the velocity profile at the end of the outlet conduit coupled to the reactor is at least 87% of the radial distance away from the inner surface thereof when the velocity of the oxygen-containing fluid is greater than or equal to 100 m/s.
24. The process according to any one of paragraphs 1 to 20, wherein the velocity maxima of the velocity profile at the end of the outlet conduit coupled to the reactor is at least 90% of the radial distance away from the inner surface thereof when the velocity of the oxygen-containing fluid is greater than or equal to 100 m/s.
25. The process according to any one of paragraphs 1 to 20, wherein the velocity maxima of the velocity profile at the end of the outlet conduit coupled to the reactor is at least 92% of the radial distance away from the inner surface thereof when the velocity of the oxygen-containing fluid is greater than or equal to 100 m/s.
26. The process according to any one of paragraphs 1 to 20, wherein the velocity maxima of the velocity profile at the end of the outlet conduit coupled to the reactor is at least 94% of the radial distance away from the inner surface thereof when the velocity of the oxygen-containing fluid is greater than or equal to 100 m/s.
27. The process according to any one of paragraphs 1 to 20, wherein the velocity maxima of the velocity profile at the end of the outlet conduit coupled to the reactor is at least 80% of the radial distance away from the inner surface thereof when the velocity of the oxygen-containing fluid is greater than or equal to 150 m/s.
28. The process according to any one of paragraphs 1 to 20, wherein the velocity maxima of the velocity profile at the end of the outlet conduit coupled to the reactor is at least 83% of the radial distance away from the inner surface thereof when the velocity of the oxygen-containing fluid is greater than or equal to 150 m/s.
29. The process according to any one of paragraphs 1 to 20, wherein the velocity maxima of the velocity profile at the end of the outlet conduit coupled to the reactor is at least 85% of the radial distance away from the inner surface thereof when the velocity of the oxygen-containing fluid is greater than or equal to 150 m/s.
30. The process according to any one of paragraphs 1 to 20, wherein the velocity maxima of the velocity profile at the end of the outlet conduit coupled to the reactor is at least 87% of the radial distance away from the inner surface thereof when the velocity of the oxygen-containing fluid is greater than or equal to 150 m/s.
31. The process according to any one of paragraphs 1 to 20, wherein the velocity maxima of the velocity profile at the end of the outlet conduit coupled to the reactor is at least 90% of the radial distance away from the inner surface thereof when the velocity of the oxygen-containing fluid is greater than or equal to 150 m/s.
32. The process according to any one of paragraphs 1 to 20, wherein the velocity maxima of the velocity profile at the end of the outlet conduit coupled to the reactor is at least 92% of the radial distance away from the inner surface thereof when the velocity of the oxygen-containing fluid is greater than or equal to 150 m/s.
33. The process according to any one of paragraphs 1 to 20, wherein the velocity maxima of the velocity profile at the end of the outlet conduit coupled to the reactor is at least 94% of the radial distance away from the inner surface thereof when the velocity of the oxygen-containing fluid is greater than or equal to 150 m/s.
34. The process according to any one of paragraphs 1 to 20, wherein the velocity maxima of the velocity profile at the end of the outlet conduit coupled to the reactor is at least 80% of the radial distance away from the inner surface thereof when the velocity of the oxygen-containing fluid is greater than or equal to 175 m/s.
35. The process according to any one of paragraphs 1 to 20, wherein the velocity maxima of the velocity profile at the end of the outlet conduit coupled to the reactor is at least 83% of the radial distance away from the inner surface thereof when the velocity of the oxygen-containing fluid is greater than or equal to 175 m/s.
36. The process according to any one of paragraphs 1 to 20, wherein the velocity maxima of the velocity profile at the end of the outlet conduit coupled to the reactor is at least 85% of the radial distance away from the inner surface thereof when the velocity of the oxygen-containing fluid is greater than or equal to 175 m/s.
37. The process according to any one of paragraphs 1 to 20, wherein the velocity maxima of the velocity profile at the end of the outlet conduit coupled to the reactor is at least 87% of the radial distance away from the inner surface thereof when the velocity of the oxygen-containing fluid is greater than or equal to 175 m/s.
38. The process according to any one of paragraphs 1 to 20, wherein the velocity maxima of the velocity profile at the end of the outlet conduit coupled to the reactor is at least 90% of the radial distance away from the inner surface thereof when the velocity of the oxygen-containing fluid is greater than or equal to 175 m/s.
39. The process according to any one of paragraphs 1 to 20, wherein the velocity maxima of the velocity profile at the end of the outlet conduit coupled to the reactor is at least 92% of the radial distance away from the inner surface thereof when the velocity of the oxygen-containing fluid is greater than or equal to 175 m/s.
40. The process according to any one of paragraphs 1 to 20, wherein the velocity maxima of the velocity profile at the end of the outlet conduit coupled to the reactor is at least 94% of the radial distance away from the inner surface thereof when the velocity of the oxygen-containing fluid is greater than or equal to 175 m/s.
41. The process according to any one of paragraphs 1 to 40, wherein the velocity of the oxygen-containing gas within the outlet conduit is about 100 m/s to about 225 m/s.
42. The process according to any one of paragraphs 1 to 40, wherein the velocity of the oxygen-containing gas within the outlet conduit is about 150 m/s to about 225 m/s.
43. The process according to any one of paragraphs 1 to 40, wherein the velocity of the oxygen-containing gas within the outlet conduit is about 175 m/s to about 225 m/s.
44. The process according to any one of paragraphs 1 to 43, wherein an amount of oxygen-containing fluid flowing through the outlet conduit is about 300 m³/s to about 400 m³/s.
45. The process according to any one of paragraphs 1 to 43, wherein the oxygen-containing fluid flowing through the outlet conduit is at a pressure of about 101 kPa to about 200 kPa.
46. The process according to any one of paragraphs 2 to 45, wherein the computational fluid dynamics modeling uses an Ansys CFX V17.2 computational fluid dynamics solver and an Ansys Mesher V17.2 meshing solver.
47. The process according to paragraph 46, wherein a mesh type used in the computational fluid dynamics modeling is fully tetrahedral with wall inflation.
48. The process according to paragraph 46 or 47, wherein a size function refinement used in the computational fluid dynamics modeling is proximity and curvature.
49. The process according to any one of paragraphs 46 to 48, wherein a number of elements used in the computational fluid dynamics modeling is greater than 1800000.
50. The process according to any one of paragraphs 46 to 49, wherein a number of wall inflation layers used in the computational fluid dynamics modeling is seven.
51. The process according to any one of paragraphs 46 to 50, wherein a maximum face size is about 0.0800D, and wherein D is a diameter of the outlet conduit at the end of the outlet conduit coupled to the reactor.
52. The process according to any one of paragraphs 46 to 51, wherein a minimum face size is about 0.0123D, and wherein D is a diameter of the outlet conduit at the end of the outlet conduit coupled to the reactor.
53. The process according to any one of paragraphs 46 to 52, wherein a maximum tetrahedral size is about 0.0984D, and wherein D is a diameter of the outlet conduit at the end of the outlet conduit coupled to the reactor.
54. A process for regenerating a coked catalyst in a reactor, comprising: flowing an oxygen-containing fluid through a first inlet conduit into a junction of an inlet assembly, wherein a second inlet conduit and an outlet conduit are coupled to the junction, wherein there is an acute angle between a longitudinal axis of the second inlet conduit and a longitudinal axis of the first inlet conduit, and wherein there is an obtuse angle between the longitudinal axis of the second inlet conduit and a longitudinal axis of the outlet conduit; flowing the oxygen-containing fluid through the junction and into the outlet conduit; flowing the oxygen-containing fluid through the outlet conduit and into a reactor coupled to an end of the outlet conduit, wherein the oxygen-containing fluid has a velocity profile at the end of the outlet conduit coupled to the reactor, wherein the end of the outlet conduit coupled to the reactor has a radial distance from an inner surface thereof to a center thereof, wherein a velocity maxima of the velocity profile at the end of the outlet conduit coupled to the reactor is at least 80% of the radial distance away from the inner surface of the outlet conduit when a velocity of the oxygen-containing fluid is greater than or equal to 100 m/s, and wherein the reactor comprises a coked catalyst comprising coke disposed on a catalyst; and combusting at least a portion of the coke disposed on the catalyst in the presence of the oxygen-containing fluid to produce a regenerated catalyst.
55. A process for regenerating a coked catalyst in a reactor, comprising: flowing an oxygen-containing fluid through a first inlet conduit into a junction of an inlet assembly, wherein a second inlet conduit and an outlet conduit are coupled to the junction, wherein there is an acute angle between a longitudinal axis of the second inlet conduit and a longitudinal axis of the first inlet conduit, and wherein there is an obtuse angle between the longitudinal axis of the second inlet conduit and a longitudinal axis of the outlet conduit; flowing the oxygen-containing fluid through the junction and into the outlet conduit; flowing the oxygen-containing fluid through the outlet conduit and into a reactor coupled to an end of the outlet conduit, wherein the oxygen-containing fluid has a velocity profile at the end of the outlet conduit coupled to the reactor, wherein the end of the outlet conduit coupled to the reactor has a radial distance from an inner surface thereof to a center thereof, wherein a velocity maxima of the velocity profile at the end of the outlet conduit coupled to the reactor is at least 80% of the radial distance away from the inner surface of the outlet conduit when a velocity of the oxygen-containing fluid is greater than or equal to 100 m/s, as determined using computational fluid dynamics modeling, and wherein the reactor comprises a coked catalyst comprising coke disposed on a catalyst; and combusting at least a portion of the coke disposed on the catalyst in the presence of the oxygen-containing fluid to produce a regenerated catalyst.
56. The process of paragraph 54 or 55, wherein the velocity maxima of the velocity profile at the end of the outlet conduit coupled to the reactor is at least 85% of the radial distance away from the inner surface of the outlet conduit when the velocity of the oxygen-containing fluid is greater than or equal to 100 m/s.
57. The process of paragraph 54 or 55, wherein the velocity maxima of the velocity profile at the end of the outlet conduit coupled to the reactor is at least 90% of the radial distance away from the inner surface of the outlet conduit when the velocity of the oxygen-containing fluid is greater than or equal to 100 m/s.
58. The process of paragraph 54 or 55, wherein the velocity maxima of the velocity profile at the end of the outlet conduit coupled to the reactor is at least 94% of the radial distance away from the inner surface of the outlet conduit when the velocity of the oxygen-containing fluid is greater than or equal to 100 m/s.
59. The process of paragraph 54 or 55, wherein the velocity maxima of the velocity profile at the end of the outlet conduit coupled to the reactor is at least 85% of the radial distance away from the inner surface of the outlet conduit when the velocity of the oxygen-containing fluid is greater than or equal to 150 m/s.
60. The process of paragraph 54 or 55, wherein the velocity maxima of the velocity profile at the end of the outlet conduit coupled to the reactor is at least 90% of the radial distance away from the inner surface of the outlet conduit when the velocity of the oxygen-containing fluid is greater than or equal to 150 m/s.
61. The process of paragraph 54 or 55, wherein the velocity maxima of the velocity profile at the end of the outlet conduit coupled to the reactor is at least 94% of the radial distance away from the inner surface of the outlet conduit when the velocity of the oxygen-containing fluid is greater than or equal to 150 m/s.
62. The process of paragraph 54 or 55, wherein the velocity maxima of the velocity profile at the end of the outlet conduit coupled to the reactor is at least 85% of the radial distance away from the inner surface of the outlet conduit when the velocity of the oxygen-containing fluid is greater than or equal to 200 m/s.
63. The process of paragraph 54 or 55, wherein the velocity maxima of the velocity profile at the end of the outlet conduit coupled to the reactor is at least 90% of the radial distance away from the inner surface of the outlet conduit when the velocity of the oxygen-containing fluid is greater than or equal to 200 m/s.
64. The process of paragraph 54 or 55, wherein the velocity maxima of the velocity profile at the end of the outlet conduit coupled to the reactor is at least 94% of the radial distance away from the inner surface of the outlet conduit when the velocity of the oxygen-containing fluid is greater than or equal to 200 m/s.
65. The process according to any one of paragraphs 54 to 64, wherein the oxygen-containing fluid has a velocity profile at an end of the outlet conduit coupled to the junction, wherein the end of the outlet conduit coupled to the junction has a radial distance from an inner surface thereof to a center thereof, and wherein a velocity maxima of the velocity profile at the end of the outlet conduit coupled to the junction is at least 80% of the radial distance away from the inner surface thereof when the velocity of the oxygen-containing fluid is greater than or equal to 100 m/s.
66. The process according to any one of paragraphs 54 to 64, wherein the oxygen-containing fluid has a velocity profile at an end of the outlet conduit coupled to the junction, wherein the end of the outlet conduit coupled to the junction has a radial distance from an inner surface thereof to a center thereof, and wherein a velocity maxima of the velocity profile at the end of the outlet conduit coupled to the junction is at least 85% of the radial distance away from the inner surface thereof when the velocity of the oxygen-containing fluid is greater than or equal to 100 m/s.
67. The process according to any one of paragraphs 54 to 64, wherein the oxygen-containing fluid has a velocity profile at an end of the outlet conduit coupled to the junction, wherein the end of the outlet conduit coupled to the junction has a radial distance from an inner surface thereof to a center thereof, and wherein a velocity maxima of the velocity profile at the end of the outlet conduit coupled to the junction is at least 90% of the radial distance away from the inner surface thereof when the velocity of the oxygen-containing fluid is greater than or equal to 100 m/s.
68. The process according to any one of paragraphs 54 to 64, wherein the oxygen-containing fluid has a velocity profile at an end of the outlet conduit coupled to the junction, wherein the end of the outlet conduit coupled to the junction has a radial distance from an inner surface thereof to a center thereof, and wherein a velocity maxima of the velocity profile at the end of the outlet conduit coupled to the junction is at least 94% of the radial distance away from the inner surface thereof when the velocity of the oxygen-containing fluid is greater than or equal to 100 m/s.
69. The process according to any one of paragraphs 54 to 64, wherein the oxygen-containing fluid has a velocity profile at an end of the outlet conduit coupled to the junction, wherein the end of the outlet conduit coupled to the junction has a radial distance from an inner surface thereof to a center thereof, and wherein a velocity maxima of the velocity profile at the end of the outlet conduit coupled to the junction is at least 80% of the radial distance away from the inner surface thereof when the velocity of the oxygen-containing fluid is greater than or equal to 150 m/s.
70. The process according to any one of paragraphs 54 to 64, wherein the oxygen-containing fluid has a velocity profile at an end of the outlet conduit coupled to the junction, wherein the end of the outlet conduit coupled to the junction has a radial distance from an inner surface thereof to a center thereof, and wherein a velocity maxima of the velocity profile at the end of the outlet conduit coupled to the junction is at least 85% of the radial distance away from the inner surface thereof when the velocity of the oxygen-containing fluid is greater than or equal to 150 m/s.
71. The process according to any one of paragraphs 54 to 64, wherein the oxygen-containing fluid has a velocity profile at an end of the outlet conduit coupled to the junction, wherein the end of the outlet conduit coupled to the junction has a radial distance from an inner surface thereof to a center thereof, and wherein a velocity maxima of the velocity profile at the end of the outlet conduit coupled to the junction is at least 90% of the radial distance away from the inner surface thereof when the velocity of the oxygen-containing fluid is greater than or equal to 150 m/s.
72. The process according to any one of paragraphs 54 to 64, wherein the oxygen-containing fluid has a velocity profile at an end of the outlet conduit coupled to the junction, wherein the end of the outlet conduit coupled to the junction has a radial distance from an inner surface thereof to a center thereof, and wherein a velocity maxima of the velocity profile at the end of the outlet conduit coupled to the junction is at least 94% of the radial distance away from the inner surface thereof when the velocity of the oxygen-containing fluid is greater than or equal to 150 m/s.
73. The process according to any one of paragraphs 54 to 64, wherein the oxygen-containing fluid has a velocity profile at an end of the outlet conduit coupled to the junction, wherein the end of the outlet conduit coupled to the junction has a radial distance from an inner surface thereof to a center thereof, and wherein a velocity maxima of the velocity profile at the end of the outlet conduit coupled to the junction is at least 85% of the radial distance away from the inner surface thereof when the velocity of the oxygen-containing fluid is greater than or equal to 200 m/s.
74. The process according to any one of paragraphs 54 to 64, wherein the oxygen-containing fluid has a velocity profile at an end of the outlet conduit coupled to the junction, wherein the end of the outlet conduit coupled to the junction has a radial distance from an inner surface thereof to a center thereof, and wherein a velocity maxima of the velocity profile at the end of the outlet conduit coupled to the junction is at least 90% of the radial distance away from the inner surface thereof when the velocity of the oxygen-containing fluid is greater than or equal to 200 m/s.
75. The process according to any one of paragraphs 54 to 64, wherein the oxygen-containing fluid has a velocity profile at an end of the outlet conduit coupled to the junction, wherein the end of the outlet conduit coupled to the junction has a radial distance from an inner surface thereof to a center thereof, and wherein a velocity maxima of the velocity profile at the end of the outlet conduit coupled to the junction is at least 94% of the radial distance away from the inner surface thereof when the velocity of the oxygen-containing fluid is greater than or equal to 200 m/s.
76. The process according to any one of paragraphs 54 to 75, wherein the velocity of the oxygen-containing fluid within the outlet conduit is about 100 m/s to about 225 m/s.
77. The process according to any one of paragraphs 54 to 75, wherein the velocity of the oxygen-containing fluid within the outlet conduit is about 150 m/s to about 225 m/s.
78. The process according to any one of paragraphs 54 to 75, wherein the velocity of the oxygen-containing fluid within the outlet conduit is about 175 m/s to about 225 m/s.
79. The process according to any one of paragraphs 54 to 75, wherein an amount of oxygen-containing fluid flowing through the outlet conduit is about 300 m³/s to about 400 m³/s.
80. The process according to any one of paragraphs 54 to 75, wherein the oxygen-containing fluid flowing through the outlet conduit is at a pressure of about 101 kPa to about 200 kPa.
81. The process according to any one of paragraphs 55 to 80, wherein the computational fluid dynamics modeling uses an Ansys CFX V17.2 computational fluid dynamics solver and an Ansys Mesher V17.2 meshing solver.
82. The process according to paragraph 81, wherein a mesh type used in the computational fluid dynamics modeling is fully tetrahedral with wall inflation.
83. The process according to paragraph 81 or 82, wherein a size function refinement used in the computational fluid dynamics modeling is proximity and curvature.
84. The process according to any one of paragraphs 81 to 83, wherein a number of elements used in the computational fluid dynamics modeling is greater than 1800000.
85. The process according to any one of paragraphs 81 to 84, wherein a number of wall inflation layers used in the computational fluid dynamics modeling is seven.
86. The process according to any one of paragraphs 81 to 85, wherein a maximum face size is about 0.0800D, and wherein D is a diameter of the outlet conduit at the end of the outlet conduit coupled to the reactor.
87. The process according to any one of paragraphs 81 to 86, wherein a minimum face size is about 0.0123D, and wherein D is a diameter of the outlet conduit at the end of the outlet conduit coupled to the reactor.
88. The process according to any one of paragraphs 81 to 87, wherein a maximum tetrahedral size is about 0.0984D, and wherein D is a diameter of the outlet conduit at the end of the outlet conduit coupled to the reactor.
89. The process according to any one of paragraphs 54 to 88, wherein the first inlet conduit comprises a pre-distributor disposed therein.
90. The process according to any one of paragraphs 54 to 88, wherein the first inlet conduit comprises a pre-distributor disposed on an inner surface thereof.
91. The process of paragraph 89 or 90, wherein the pre-distributor comprises a band of material disposed on an inner surface of the first inlet conduit.
92. The process of paragraph 91, wherein the band of material has a substantially symmetrical inner surface.
93. The process of paragraph 91, wherein the band of material comprises a ring having a substantially constant width.
94. The process of paragraph 91, wherein the band of material comprises a ring having a plurality of tabs attached to an inner surface thereof, wherein the plurality of tabs is positioned to provide the band of material with a substantially symmetrical inner surface.
95. The process of paragraph 32, wherein each tab has a geometrical shape selected from the group consisting of: a semi-rectangle and a semi-ellipse.
96. The process of paragraph 89 or 90, wherein the pre-distributor comprises a plurality of discrete tabs disposed on an inner surface of the first inlet conduit.
97. The process of paragraph 97, wherein a location of each of the plurality of discrete tabs is substantially the same along the longitudinal axis of the first inlet conduit with respect to one another.
98. The process of paragraph 96 or 97, wherein the plurality of discrete tabs provide a substantially symmetrical inner surface.
99. The process of paragraph 89 or 90, wherein the pre-distributor comprises a plurality of rings disposed on an inner surface of the first inlet conduit.
100. A process for introducing a gas into a reactor, comprising: flowing a gas through a first inlet conduit into a junction of an inlet assembly, wherein a second inlet conduit and an outlet conduit are coupled to the junction, wherein there is an acute angle between a longitudinal axis of the second inlet conduit and a longitudinal axis of the first inlet conduit, and wherein there is an obtuse angle between the longitudinal axis of the second inlet conduit and a longitudinal axis of the outlet conduit; flowing the gas through the junction and into the outlet conduit; and flowing the gas into a reactor through an end of the outlet conduit, wherein the gas has a velocity profile at the end of the outlet conduit, wherein the end of the outlet conduit has a radial distance from an inner surface thereof to a center thereof, and wherein a velocity maxima of the velocity profile at the end of the outlet conduit is at least 80% of the radial distance away from the inner surface of the end of the outlet conduit when a velocity of the gas is greater than or equal to 100 m/s.
101. The process of paragraph 100, wherein the velocity maxima of the velocity profile at the end of the outlet conduit is at least 85%, at least 90%, at least 92%, or at least 94% of the radial distance away from the inner surface of the end of the outlet conduit when the velocity of the gas is greater than or equal to 100 m/s.
102. The process of paragraph 100, wherein the velocity maxima of the velocity profile at the end of the outlet conduit is at least 80%, at least 85%, at least 90%, at least 92%, or at least 94% of the radial distance away from the inner surface of the end of the outlet conduit when the velocity of the gas is greater than or equal to 150 m/s.
103. The process of paragraph 100, wherein the velocity maxima of the velocity profile at the end of the outlet conduit is at least 80%, at least 85%, at least 90%, at least 92%, or at least 94% of the radial distance away from the inner surface of the end of the outlet conduit when the velocity of the gas is greater than or equal to 200 m/s.
104. An inlet assembly for introducing a fluid into a reactor, comprising: a first inlet conduit, a second inlet conduit, and an outlet conduit fluidly connected at a junction, wherein the first inlet conduit is configured to convey a first gas therethrough, the second inlet conduit is configured to convey a second gas therethrough, and the outlet conduit is configured to convey the first gas and the second gas therethrough and into a reactor, wherein there is an acute angle between a longitudinal axes of the first inlet conduit and a longitudinal axis of the second inlet conduit and an obtuse angle between a longitudinal axis of the outlet conduit and the longitudinal axis of the second inlet conduit; and a pre-distributor disposed within the first inlet conduit.
105. The inlet assembly of paragraph 104, wherein a cross-sectional area of the first inlet conduit at the junction is greater than a cross-sectional area of the second inlet conduit at the junction.
106. The inlet assembly of paragraph 104 or 105, further comprising a third inlet conduit fluidly connected to the junction and configured to introduce least one fluid selected from the group consisting of: a reducing gas, a purge gas, and steam, and wherein there is an acute angle between the longitudinal axis of the first conduit and a longitudinal axis of the third conduit and an obtuse angle between the longitudinal axis of the inlet and the longitudinal axis of the third conduit.
107. The inlet assembly of paragraph 106, wherein a cross-sectional area of the first conduit at the junction is greater than a cross-sectional area of the second conduit at the junction, and wherein the cross-sectional area of the second conduit at the junction is greater than a cross-sectional area of the third conduit at the junction.
108. The inlet assembly according to any one of claims 104 to 107, wherein the longitudinal axis of the first inlet conduit and the longitudinal axis of the outlet conduit are axially aligned with one another.
109. The inlet assembly according to any one of paragraphs 104 to 108, wherein the pre-distributor is disposed on an inner surface of the first conduit.
110. The inlet assembly according to any one of claims 104 to 109, wherein the pre-distributor comprises a band of material disposed on an inner surface of the first conduit.
111. The inlet assembly of paragraph 110, wherein the band of material is disposed about an inner perimeter of the first conduit.
112. The inlet assembly of paragraph 110, wherein the band of material has a substantially symmetrical inner surface.
113. The inlet assembly of paragraph 110, wherein the band of material comprises a ring having a substantially constant width.
114. The inlet assembly of paragraph 110, wherein the band of material comprises a ring having a plurality of tabs attached to an inner surface thereof.
115. The inlet assembly of paragraph 114, wherein the plurality of tabs is positioned to provide the band of material with a substantially symmetrical inner surface.
116. The inlet assembly of paragraph 114 or 115, wherein each tab has a geometrical shape selected from the group consisting of: a semi-rectangle and a semi-ellipse.
117. The inlet assembly according to any one of paragraphs 104 to 108, wherein the pre-distributor comprises a plurality of discrete tabs disposed on an inner surface of the first conduit.
118. The inlet assembly of paragraph 117, wherein a location of each of the plurality of discrete tabs along the longitudinal axis of the first conduit is substantially the same with respect to one another.
119. The inlet assembly of paragraph 117 or 118, wherein the plurality of discrete tabs provide a substantially symmetrical inner surface.
120. The inlet assembly according to any one of paragraphs 104 to 107, wherein the pre-distributor comprises a plurality of rings disposed on an inner surface of the first conduit.
121. An inlet assembly for introducing a fluid into a reactor, comprising: a first inlet conduit, a second inlet conduit, and an outlet conduit fluidly connected at a junction, wherein the first inlet conduit is configured to convey a first gas therethrough, the second inlet conduit is configured to convey a second gas therethrough, and the outlet conduit is configured to convey the first gas and the second gas therethrough and into a reactor, wherein there is an acute angle between a longitudinal axes of the first inlet conduit and a longitudinal axis of the second inlet conduit and an obtuse angle between a longitudinal axis of the outlet conduit and the longitudinal axis of the second inlet conduit; and a pre-distributor disposed on an inner surface of the first inlet conduit.
122. The inlet assembly of paragraph 121, wherein a cross-sectional area of the first inlet conduit at the junction is greater than a cross-sectional area of the second inlet conduit at the junction.
123. The inlet assembly of paragraph 121 or 122, further comprising a third inlet conduit fluidly connected to the junction and configured to introduce least one fluid selected from the group consisting of: a reducing gas, a purge gas, and steam, and wherein there is an acute angle between the longitudinal axis of the first conduit and a longitudinal axis of the third conduit and an obtuse angle between the longitudinal axis of the inlet and the longitudinal axis of the third conduit.
124. The inlet assembly of paragraph 123, wherein a cross-sectional area of the first conduit at the junction is greater than a cross-sectional area of the second conduit at the junction, and wherein the cross-sectional area of the second conduit at the junction is greater than a cross-sectional area of the third conduit at the junction.
125. The inlet assembly according to any one of paragraphs 121 to 124, wherein the longitudinal axis of the first inlet conduit and the longitudinal axis of the outlet conduit are axially aligned with one another.
126. The inlet assembly according to any one of paragraphs 121 to 125, wherein the pre-distributor comprises a band of material disposed on an inner surface of the first conduit.
127. The inlet assembly according to any one of paragraphs 121 to 125, wherein the pre-distributor comprises a ring having a plurality of tabs attached to an inner surface thereof.
128. The inlet assembly according to any one of paragraphs 121 to 125, wherein the pre-distributor comprises a ring.
129. The inlet assembly according to any one of paragraphs 121 to 125, wherein the pre-distributor comprises a ring having a substantially constant width.
130. The inlet assembly according to any one of paragraphs 121 to 125, wherein the pre-distributor comprises a plurality of tabs.
131. The process according to any one of paragraphs 54 to 99, wherein the oxygen containing fluid flowing through the first inlet conduit is in the turbulent flow regime.
132. The process according to any one of paragraphs 54 to 99 and 130, wherein the oxygen containing fluid flowing through the junction is in the turbulent flow regime.
133. The process according to any one of paragraphs 54 to 99, 130, and 132, wherein the oxygen containing fluid flowing through the outlet conduit is in the turbulent flow regime.
134. The process according to any one of paragraphs 100 to 103, wherein the gas flowing through the first inlet conduit is in the turbulent flow regime.
135. The process according to any one of paragraphs 100 to 103 and 134, wherein the gas flowing through the junction is in the turbulent flow regime.
136. The process according to any one of paragraphs 100 to 103, 133, and 135, wherein the gas flowing through the outlet conduit is in the turbulent flow regime.

Certain embodiments and features have been described using a set of numerical upper limits and a set of numerical lower limits. It should be appreciated that ranges including the combination of any two values, *e.g.,* the combination of any lower value with any upper value, the combination of any two lower values, and/or the combination of any two upper values are contemplated unless otherwise indicated. Certain lower limits, upper limits and ranges appear in one or more claims below. All numerical values are "about" or "approximately" the indicated value, and take into account experimental error and variations that would be expected by a person having ordinary skill in the art.

Various terms have been defined above. To the extent a term used in a claim is not defined above, it should be given the broadest definition persons in the pertinent art have given that term as reflected in at least one printed publication or issued patent. And if applicable, all patents, test procedures, and other documents cited in this application are fully incorporated by reference to the extent such disclosure is not inconsistent with this application and for all jurisdictions in which such incorporation is permitted.

While the foregoing is directed to certain illustrative embodiments, other and further embodiments of the invention can be devised without departing from the basic scope thereof, and the scope thereof is determined by the claims that follow.

## Claims

1. An inlet assembly for introducing a fluid into a reactor, comprising:
a first inlet conduit, a second inlet conduit, and an outlet conduit fluidly connected at a junction, wherein the first inlet conduit is configured to convey a first gas therethrough, the second inlet conduit is configured to convey a second gas therethrough, and the outlet conduit is configured to convey the first gas and the second gas therethrough and into a reactor, wherein there is an acute angle between a longitudinal axes of the first inlet conduit and a longitudinal axis of the second inlet conduit and an obtuse angle between a longitudinal axis of the outlet conduit and the longitudinal axis of the second inlet conduit; and
a pre-distributor disposed within the first inlet conduit.

2. The inlet assembly of claim 1, wherein a cross-sectional area of the first inlet conduit at the junction is greater than a cross-sectional area of the second inlet conduit at the junction.

3. The inlet assembly of claim 1, further comprising a third inlet conduit fluidly connected to the junction and configured to introduce at least one fluid selected from the group consisting of: a reducing gas, a purge gas, and steam, and wherein there is an acute angle between the longitudinal axis of the first conduit and a longitudinal axis of the third conduit and an obtuse angle between the longitudinal axis of the inlet and the longitudinal axis of the third conduit.

4. The inlet assembly of claim 3, wherein a cross-sectional area of the first conduit at the junction is greater than a cross-sectional area of the second conduit at the junction, and wherein the cross-sectional area of the second conduit at the junction is greater than a cross-sectional area of the third conduit at the junction.

5. The inlet assembly according to claim 1, wherein the longitudinal axis of the first inlet conduit and the longitudinal axis of the outlet conduit are axially aligned with one another.

6. The inlet assembly according to claim 1, wherein the pre-distributor is disposed on an inner surface of the first conduit.

7. The inlet assembly according to claim 1, wherein the pre-distributor comprises a band of material disposed on an inner surface of the first conduit.

8. The inlet assembly of claim 7, wherein the band of material
is disposed about an inner perimeter of the first conduit, and/or
has a substantially symmetrical inner surface, and/or
comprises a ring having a substantially constant width.

9. The inlet assembly of claim 7, wherein the band of material comprises a ring having a plurality of tabs attached to an inner surface thereof.

10. The inlet assembly of claim 9, wherein the plurality of tabs is positioned to provide the band of material with a substantially symmetrical inner surface.

11. The inlet assembly of claim 9, wherein each tab has a geometrical shape selected from the group consisting of a semi-rectangle and a semi-ellipse.

12. The inlet assembly according to claim 1, wherein the pre-distributor comprises a plurality of discrete tabs disposed on an inner surface of the first conduit.

13. The inlet assembly of claim 12, wherein a location of each of the plurality of discrete tabs along the longitudinal axis of the first conduit is substantially the same with respect to one another,
wherein the plurality of discrete tabs preferably provide a substantially symmetrical inner surface.

14. The inlet assembly according to claim 1, wherein the pre-distributor comprises a plurality of rings disposed on an inner surface of the first conduit.

15. An inlet assembly according to one of claims 1 to 3, wherein the pre-distributor is disposed on an inner surface of the first inlet conduit.
